(19) **Europäisches Patentamt** / **European Patent Office** / **Office européen des brevets**

(11) **EP 3 962 560 B1**

(12) **EUROPEAN PATENT SPECIFICATION**

(45) Date of publication and mention
of the grant of the patent:
**25.06.2025 Bulletin 2025/26**

(21) Application number: **20798622.5**

(22) Date of filing: **01.05.2020**

(51) International Patent Classification (IPC):
***A61M 16/00*** *(2006.01)*

(52) Cooperative Patent Classification (CPC):
**A61M 16/024; A61B 5/087; A61B 5/7257;
A61M 16/0051; A61M 16/0066; A61M 16/06;**
A61B 7/003; A61M 16/026; A61M 16/0875;
A61M 16/16; A61M 2016/0027; A61M 2016/003;
A61M 2016/0036; A61M 2205/13; A61M 2205/3365;
(Cont.)

(86) International application number:
**PCT/AU2020/050435**

(87) International publication number:
**WO 2020/220091 (05.11.2020 Gazette 2020/45)**

(54) **ACOUSTIC COMPONENT IDENTIFICATION FOR RESPIRATORY THERAPY SYSTEMS**

AKUSTISCHE KOMPONENTENIDENTIFIZIERUNG FÜR ATEMTHERAPIESYSTEME

IDENTIFICATION DE COMPOSANT ACOUSTIQUE POUR SYSTÈMES DE THÉRAPIE RESPIRATOIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **02.05.2019 AU 2019901502**

(43) Date of publication of application:
**09.03.2022 Bulletin 2022/10**

(60) Divisional application:
**22181561.6 / 4 119 047**

(73) Proprietor: **ResMed Pty Ltd
Bella Vista, New South Wales 2153 (AU)**

(72) Inventor: **HOLLEY, Liam
Bella Vista, New South Wales 2153 (AU)**

(74) Representative: **Vossius & Partner
Patentanwälte Rechtsanwälte mbB
Siebertstraße 3
81675 München (DE)**

(56) References cited:
WO-A1-2010/091462   WO-A1-2019/006496
WO-A1-2019/070135   WO-A1-2020/024914
WO-A2-2006/107624   AU-A1- 2013 201 312
AU-A1- 2014 271 343   US-A1- 2011 313 689
US-A1- 2019 126 000   US-A1- 2019 232 014

• **VASEGHI S. V., ADVANCED DIGITAL SIGNAL PROCESSING AND NOISE REDUCTION, 2000, pages 333 - 336, XP055758261, ISBN: 0-471-62692-9**

**(Cont. next page)**

(52) Cooperative Patent Classification (CPC): (Cont.)
A61M 2205/3375; A61M 2205/3553;
A61M 2205/3592; A61M 2205/42; A61M 2205/52;
A61M 2205/60; A61M 2205/6018; A61M 2205/70

**Description**

**1 CROSS-REFERENCE TO RELATED APPLICATIONS**

This application claims the benefit of Australian Provisional Application No. 2019901502, filed 2 May 2019.

**2 BACKGROUND OF THE TECHNOLOGY**

**2.1 FIELD OF THE TECHNOLOGY**

The present technology relates to a device for generating a respiratory therapy. For example, devices of the present technology may implement acoustic techniques such as for component identification and/or control of such devices for generation of therapy.

**2.2 DESCRIPTION OF THE RELATED ART**

*2.2.1 Human Respiratory System and its Disorders*

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"Respiratory Physiology"*, by John B. West, Lippincott Williams & Wilkins, 9th edition published 2012.

A range of respiratory disorders exist. Certain disorders may be characterised by particular events, e.g. apneas, hypopneas, and hyperpneas.

Obstructive Sleep Apnea (OSA) is a respiratory disorder characterised by events including occlusion or obstruction of the upper air passage during sleep. It results from a combination of an abnormally small upper airway and the normal loss of muscle tone in the region of the tongue, soft palate and posterior oropharyngeal wall during sleep. The condition causes the affected patient to stop breathing for periods known as apneas, typically of 30 to 120 seconds in duration, sometimes 200 to 300 times per night. It often causes excessive daytime somnolence, and it may cause cardiovascular disease and brain damage. The syndrome is a common disorder, particularly in middle aged overweight males, although a person affected may have no awareness of the problem. See US Patent No. 4,944,310 (Sullivan).

A range of therapies have been used to treat or ameliorate such conditions. Furthermore, otherwise healthy individuals may take advantage of such therapies to prevent respiratory disorders from arising. However, these have a number of shortcomings.

*2.2.2 Therapies*

Various therapies, such as Continuous Positive Airway Pressure (CPAP) therapy, high flow therapy (HFT), non-invasive ventilation (NIV) and invasive ventilation (IV) have been used to treat one or more of the above respiratory disorders.

*2.2.3 Therapy Systems*

These therapies may be provided by a respiratory therapy system or device. Such systems and devices may also be used to diagnose a condition without treating it.

A respiratory therapy system may comprise a Respiratory Therapy Device (RT device), an air circuit, a humidifier, a patient interface, and data management.

*2.2.3.1 Patient Interface*

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 $cmH_2O$ relative to ambient pressure. For other forms of therapy, such as the delivery of oxygen, the patient interface may not include a seal sufficient to facilitate delivery to the airways of a supply of gas at a positive pressure of about 10 $cmH_2O$.

*2.2.3.2 Respiratory Therapy (RT) Device*

A respiratory therapy (RT) device, such as a respiratory pressure therapy (RPT) device, may be used to deliver one or more of a number of therapies described above, such as by generating a flow of air for delivery to an entrance to the airways. The flow of air may be pressurised. Examples of RPT devices include a CPAP device and a ventilator. A respiratory therapy (RT) device in some cases may be a high flow therapy (HFT) device, which provides a high flow respiratory therapy.

Air pressure generators are known in a range of applications, e.g. industrial-scale ventilation systems. However, air pressure generators for medical applications have particular requirements not fulfilled by more generalised air pressure generators, such as the reliability, size and weight requirements of medical devices.

Examples of RPT devices include the S9 Sleep Therapy System, manufactured by ResMed Limited, ventilators such as the ResMed Stellar™ Series of Adult and Paediatric Ventilators and the ResMed Astral™ 150 ventilator.

*2.2.3.3 Humidifier*

Delivery of a flow of air without humidification may cause drying of airways. The use of a humidifier with an RT device and the patient interface produces humidified gas that minimizes drying of the nasal mucosa and increases patient airway comfort. In addition, in cooler climates, warm air applied generally to the face area in and about the patient interface is more comfortable than cold air.

*2.2.3.4 Vent technologies*

Some forms of respiratory therapy systems may include a vent to allow the washout of exhaled carbon dioxide. The vent may allow a flow of gas from an interior space of a patient interface, e.g., the plenum chamber, to an exterior of the patient interface, e.g., to ambient.

*2.2.3.5 Sensing and Data Management*

A patient, a caregiver, a clinician, an insurance company or a technician may wish to collect data in relation to a respiratory therapy, whether they relate to the patient, individual components used for therapy, or the therapy system as a whole. There exist numerous situations in providing respiratory therapy to a patient where one or more parties involved therein may benefit from collection of therapy related data, and leveraging the collected data.

In particular, some components of a respiratory therapy system need to be replaced at a higher frequency than others for effective therapy. For example, a patient interface comprising a silicone seal-forming portion may be replaced by some patients in periods of months (e.g. 3 months), whereas RT devices may be replaced or upgraded every few years (e.g. 3 years). For those components that are to be replaced at relatively frequent intervals (e.g. a patient interface), a patient or caregiver regularly faces challenges in being reliably and accurately notified, at a low cost, when their component is due to be replaced. When it is replaced, the new component may necessitate the patient or caregiver to change one or more settings in the therapy system (e.g. a software setting in the RT device) to ensure that the system is taking full advantage of the new component. The ability to identify components of a respiratory therapy system is therefore important both for optimising therapy and for keeping patients and caregivers informed about replacement timing.

In the past, an array of solutions have been employed, or proposed, in the field of respiratory therapy in relation to component identification. For example, sensors/transducers have been used, and proposed, in a plethora of forms to collect data in relation to environmental conditions, information in relation to the patient, identification of components, therapy operating conditions or the like. Indeed, many RT devices comprise one or more sensors, such as a flow rate sensor, a pressure sensor, a humidity sensor, a temperature sensor and the like. Signals generated by such sensors may be analysed to generated therapy related data such as the identity of particular components, such as patient interfaces, in the respiratory therapy system.

However, sensors/transducers typically require a suite of additional componentry, which may hinder their adoption in many forms. For instance, data collected by the sensors/transducers must then be communicated to be saved and/or analysed, for example from the sensor to a memory and/or a processor. This, and the aforementioned sensors, may further increase cost of design, testing, and/or manufacturing to the medical device manufacturer, and/or may increase the cost and complexity to the patient.

In addition, integrating costly electrical and/or mechanical features to the frequently replaced component(s), such as the patient interface, may be detrimental to providing a most cost-effective therapy, and potentially environmentally unsustainable due to the increased waste.

Furthermore, many proposed solutions in relation to sensors and/or transducers may be limited in that if a sensor is proposed to be located remotely from where its data is to be saved and/or analysed, often this may further increase a complexity and/or cost of implementation. For example, where a patient interface comprises a sensor, it may require an electrical connection to the RT device, which may further increase a complexity and/or cost of implementation.

Also, designers of RT devices face numerous choices, and often arrive at different solutions when compared to other devices on the market such as by a competitor, or indeed, by the same manufacturer but produced at a different time. As a result, the associated electrical connector provided may be only connectible to a particular RT device. This may have an unintended effect of creating incompatibility which can be a disadvantage to a particular sub-segment of consumers, and/or it may reduce consumer choice.

WO 2010/091462 A1, AU 2014 271 343 A1 and AU 2013 201 312 A1 relate to methods and apparatus which provide acoustic detection for automated devices such as respiratory treatment apparatus. Acoustic analysis of noise or sound pulses, such as a cepstrum analysis, based on signals of a sound sensor permits detection of obstruction such as within a patient interface, mask or respiratory conduit or within patient respiratory system. Some embodiments further permit detection of accessories such as an identification thereof or a condition of use thereof, such as a leak. Still further embodiments of the technology permit the detection of a patient or user who is intended to use the automated device. WO 2019/006496 A1 relates to a respiratory therapy system including one or more acoustic generators to produce an inaudible acoustic signal. The acoustic generator(s), such as when coupled to a patient interface or air circuit of a respiratory therapy device, may provide inaudible acoustic signals indicative of one or more parameters, such as a flow rate or a pressure of the flow of air, or a type of or useable life of a component (e. g. patient interface). The system may have an acoustic receiver that may detect one or more acoustic signals from the acoustic generator, the RT system, the patient or the environment.

## 3 BRIEF SUMMARY OF THE TECHNOLOGY

The invention is defined by the features of the independent claim. Preferred embodiments are defined in the dependent claims.

The present technology is directed towards providing medical devices used in the diagnosis, amelioration, treatment, or prevention of respiratory disorders having one or more of improved comfort, cost, efficacy, ease of use, patient engagement, and manufacturability.

A first aspect of the present technology relates to apparatus used in the diagnosis, amelioration, treatment or prevention of a respiratory disorder.

Another aspect of the present technology relates to methods used in the diagnosis, amelioration, treatment or prevention of a respiratory disorder.

One aspect of the present technology relates to improved respiratory therapy devices configured to identify components of the respiratory therapy systems of which they are a part, by acoustic means. In particular, the disclosed devices comprise structures and processes configured to analyse acoustic reflections from system components to identify those components from their "acoustic signatures" in a more accurate manner than previously known. This improvement may at least partly be achieved by implementing one or more structures that reduce back-reflections of sounds from the device end of the air circuit, which may, for example, improve the distinguishability of acoustic signatures of different patient interface types. The improvement may also at least partly be achieved by signal processing that "flattens" a spectrum of a sound signal, such as the log spectrum, such as before converting to an acoustic signature.

Some implementations of the present technology may include a device for generating a respiratory therapy. The device may include a pressure generator configured to generate a supply of pressurized air from an outlet along an air circuit to a patient interface. The device may include a sensor configured to generate a sound signal representing a sound of the pressure generator in the air circuit. The device may include a dampening structure configured to reduce reflection of sound from the pressure generator along the air circuit. The device may include a controller. The controller may be configured to process the sound signal so as to identify the patient interface and / or the air circuit.

In some implementations, the dampening structure may be formed by a pass-through dampening conduit configured to change acoustic impedance between the air circuit and a cavity of a housing of the pressure generator. The dampening structure defines, of may defined by, a cross-section of a passage through the pass-through dampening conduit such that the cross-section expands, due to a profile of an internal surface of the pass-through dampening conduit, along a path of the passage. The cross-section gradually expands as the cross-section may be more distant from a patient interface end of the air circuit. The device may include a waveguide at least formed by the outlet and the air circuit, and wherein the dampening structure may be located along the waveguide between the sensor and the outlet, and wherein the sensor may be located along the waveguide between the dampening structure and the air circuit. The dampening structure may be formed by a horn. The horn may have a conical profile.

Some implementations of the present technology may include a method in a processor associated with a respiratory therapy device for identifying a component of an airpath coupled to the respiratory therapy device. The method may include processing a sound signal representing a sound in the airpath to obtain a cepstrum. The processing may include flattening a spectrum of the sound signal. The processing may include separating an acoustic signature from the cepstrum. The processing may include comparing the acoustic signature to a set of predetermined acoustic signatures corresponding to respective components. The processing may include identifying the component based on the comparison of the acoustic signature to the set.

In some implementations, the flattening may include removing a low-pass filtered version of a log spectrum of the sound signal from the log spectrum of the sound signal. The removing may include subtraction. The method further may include repeating the processing and separating at least once to generate multiple acoustic signatures. The method further may include combining the multiple acoustic signatures into a combined acoustic signature. The comparing may compare the combined acoustic signature with the set of predetermined acoustic signatures. The combining may include aligning one or more of the multiple acoustic signatures with the combined acoustic signature. The combining may include averaging the multiple acoustic signatures. The component may be a patient interface and the repeating may be synchronised with a breathing cycle of a patient wearing the patient interface. The combining may be robust to small variations in the delay between the multiple acoustic signatures. The method may include adjusting a control parameter for operation of a pressure generator of the respiratory therapy device based on the identifying.

Some implementations of the present technology may include a device for generating a respiratory therapy. The device may include a pressure generator configured to generate a supply of pressurized air from an outlet along an air circuit to a patient interface. The device may include a sensor configured to generate a sound signal representing a sound of the pressure generator in the air circuit. The device may include a controller. The controller may be configured to process the sound signal to obtain a cepstrum. The processing may include flattening a spectrum of the sound signal. The controller may be configured to separate an acoustic signature from the cepstrum. The controller may be configured to compare the acoustic signature to a set of predetermined acoustic signatures corresponding to respective components. The controller may be configured to identify the patient interface and / or the air circuit based on the comparison of the acoustic signature to the set.

In some implementations, the device may include a dampening structure configured to reduce reflection of sound from the pressure generator along the air circuit. The dampening structure may be formed by a pass-through dampening conduit configured to change acoustic impedance between the air circuit and a cavity of a housing of the pressure generator. The dampening structure may be formed by a horn. The horn may have a conical profile. The controller may be further configured to adjust a control parameter for operation of the pressure generator based on the identified patient interface and/or air circuit.

Some implementations of the present technology may include a method in a processor associated with a respiratory therapy device for identifying a component of an airpath coupled to the respiratory therapy device. The method may include processing a sound signal representing a sound in the airpath to obtain a cepstrum. The method may include separating an acoustic signature from the cepstrum. The method may include repeating the processing and separating at least once to generate a plurality of acoustic signatures. The method may include combining the plurality of acoustic signatures into a combined acoustic signature. The method may include comparing the combined acoustic signature to a set of predetermined acoustic signatures corresponding to respective components. The method may include identifying the component based on the comparison of the acoustic signature to the set.

In some implementations, the combining may include aligning one or more of the plurality of acoustic signatures with the combined acoustic signature. The combining may include averaging of the plurality of acoustic signatures. The component may be a patient interface. The repeating may be synchronised with a breathing cycle of a patient wearing the patient interface. The combining may be robust to variations in the delay between the plurality of acoustic signatures. The processing may include flattening a spectrum of the sound signal. The flattening may include subtracting a low-pass filtered version of a log spectrum of the sound signal from the log spectrum of the sound signal. The method may include adjusting a control parameter for operation of a pressure generator of the respiratory therapy device based on the identifying.

Some implementations of the present technology may include a computer-readable medium having encoded thereon computer-readable instructions that when executed by a processor of a controller of a respiratory therapy device cause the processor to perform the method(s), or any one or more of the aspects thereof, as described herein.

Some implementations of the present technology may include a device for generating a respiratory therapy. The device may include a pressure generator configured to generate a supply of pressurized air from an outlet along an air circuit to a patient interface. The device may include a sensor configured to generate a sound signal representing a sound of the pressure generator in the air circuit. The device may include a controller. The controller may be configured to process the sound signal to obtain a cepstrum. The controller may be configured to separate an acoustic signature from the cepstrum. The controller may be configured to repeat the processing and separating at least once to generate multiple acoustic signatures. The controller may be configured to combine the multiple acoustic signatures into a combined acoustic signature. The controller may be configured to compare the combined acoustic signature to a set of predetermined acoustic signatures corresponding to respective components. The controller may be configured to identify the patient interface and / or the air circuit based on the comparison of the acoustic signature to the set.

In some implementations, the device may include a dampening structure configured to reduce reflection of sound from the pressure generator along the air circuit. The dampening structure may be formed by a pass-through dampening conduit configured to change acoustic impedance between the air circuit and a cavity of a housing of the pressure generator. The dampening structure may be formed by a horn. The horn may have a conical profile. In order to combine the multiple acoustic signatures, the controller may be configured to align one or more of the multiple acoustic signatures with the combined acoustic signature. In order to combine the multiple acoustic signatures, the controller may be configured to average the multiple acoustic signatures. The controller may be further configured to adjust a control parameter for operation of the pressure generator based on the identified patient interface and/or air circuit.

The methods, systems, devices and apparatus described herein can provide improved functioning in a processor, such as of a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

## 4 BRIEF DESCRIPTION OF THE DRAWINGS

The present technology is illustrated by way of example, and not by way of limitation, in the figures of the accompanying drawings, in which like reference numerals refer to similar elements including:

### 4.1 THERAPY SYSTEMS

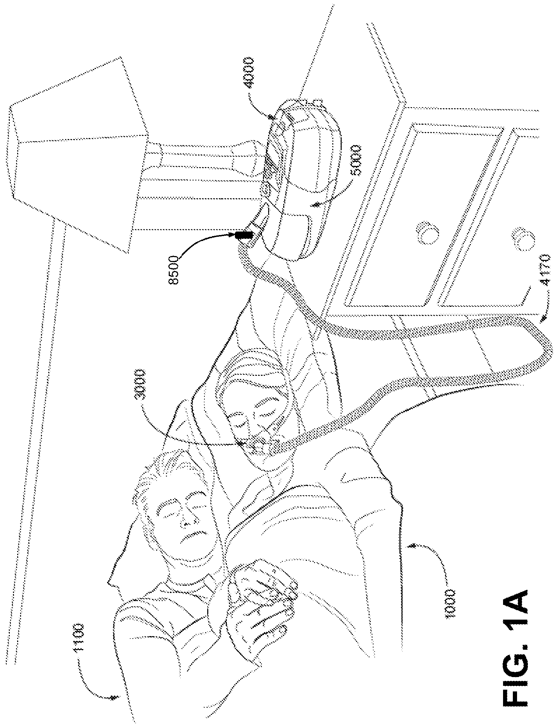

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

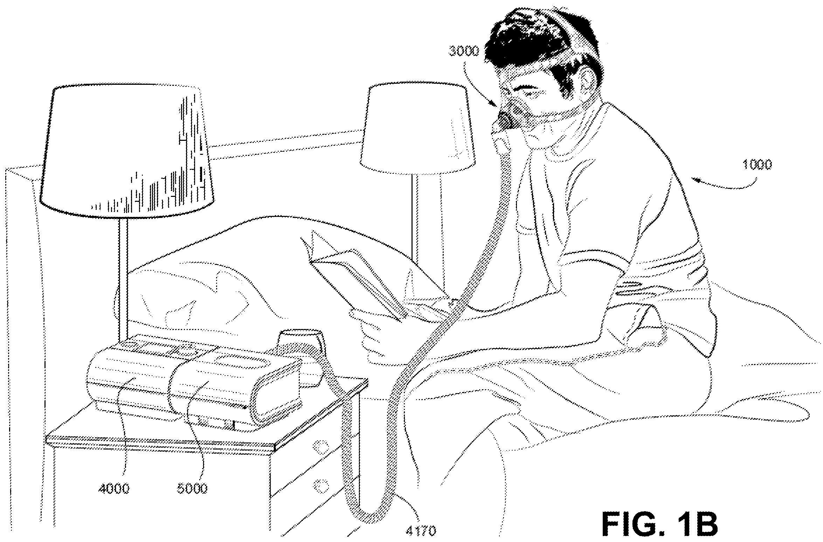

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

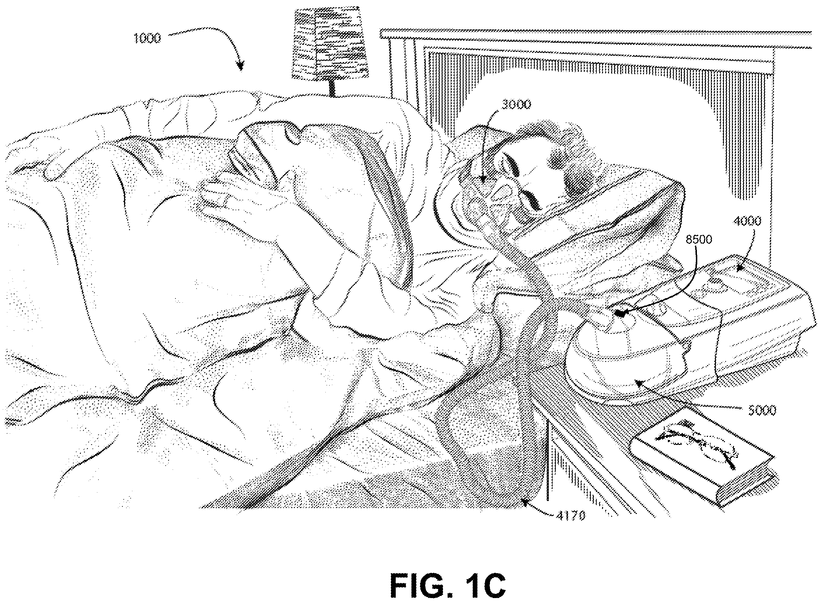

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 4.2 RESPIRATORY SYSTEM AND FACIAL ANATOMY

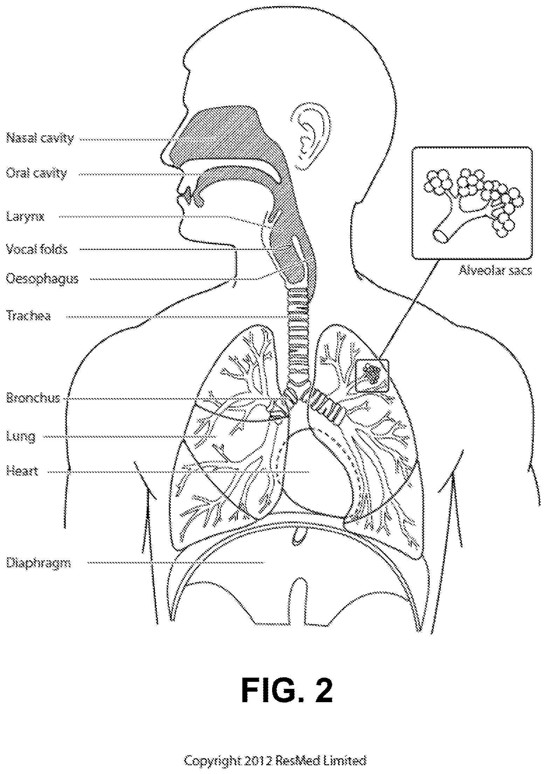

Fig. 2 shows an overview of a human respiratory system including the nasal and oral cavities, the larynx, vocal folds, oesophagus, trachea, bronchus, lung, alveolar sacs, heart and diaphragm.

### 4.3 PATIENT INTERFACE

Fig. 3 shows an example of a patient interface in the form of a nasal mask in accordance with one form of the present technology.

### 4.4 RPT DEVICE

Fig. 4A shows an exploded view of an example respiratory pressure therapy (RPT) device 4000 in accordance with one form of the present technology.

Fig. 4B is a schematic diagram of the pneumatic path of an RPT device in accordance with one form of the present technology. The directions of upstream and downstream are indicated.

### 4.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 4.6 BREATHING WAVEFORMS

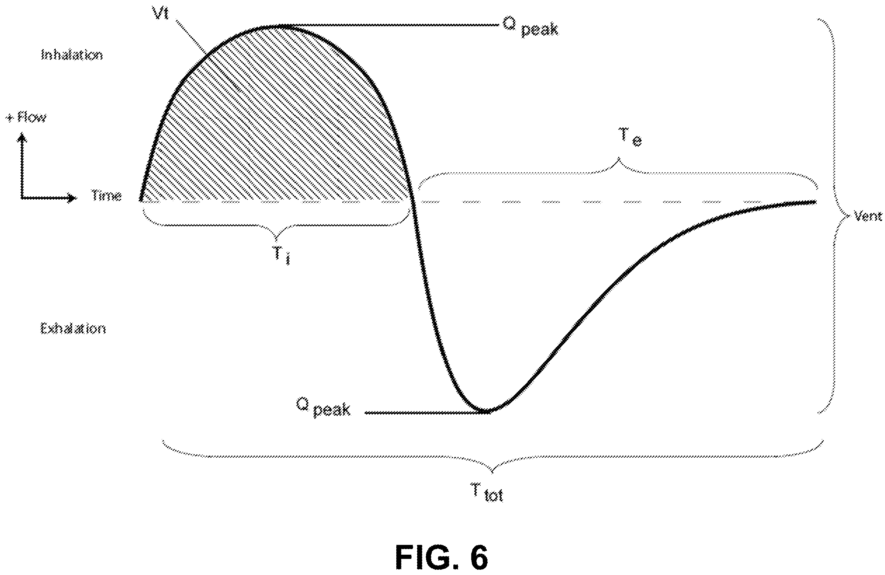

Fig. 6 shows a model typical breath waveform of a person while sleeping. The horizontal axis is time, and the vertical axis is respiratory flow rate. While the parameter values may vary, a typical breath may have the following approximate values: tidal volume, $Vt$, 0.5L, inhalation time, $Ti$, 1.6s, peak inspiratory flow rate, $Qpeak$, 0.4 L/s, exhalation time, $Te$, 2.4s, peak expiratory flow rate, $Qpeak$, -0.5 L/s. The total duration of the breath, $Ttot$, is about 4s. The person typically breathes at a rate of about 15 breaths per minute (BPM), with Ventilation, $Vent$, about 7.5 L/min. A typical duty cycle, the ratio of $Ti$ to $Ttot$, is about 40%.

### 4.7 ACOUSTIC ANALYSIS

Fig. 7 is a schematic view of a respiratory therapy system in accordance with one aspect of the present technology.

Fig. 8 is a graph containing an example of an Impulse Response Function of the respiratory therapy system of Fig. 7;

Fig. 9 is a graph containing cepstra of various example masks at various blower speeds in the respiratory therapy system of Fig. 7;

Fig. 10 is a graph containing a cepstrum containing a back-reflection component;

Fig. 11 is a schematic view of a respiratory therapy system in accordance with one aspect of the present technology;

Figs. 11A and 11B illustrate profiles for example pass-through dampening conduits for some implementations of the present technology;

Fig. 12 is a flow chart illustrating a method of identifying a component of the airpath of a respiratory therapy system in accordance with one aspect of the present technology;

Fig. 13 contains two graphs illustrating acoustic signatures before and after alignment.

### 5 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.
The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 5.1 THERAPY

In one form, the present technology comprises a method for treating a respiratory disorder comprising the step of applying positive pressure to the entrance of the airways of a patient 1000.

## 5.2 THERAPY SYSTEMS

In one form, the present technology comprises a system for treating a respiratory disorder. The respiratory therapy (RT) system may comprise an RPT device 4000 for delivering a supply of air at positive pressure to the patient 1000 via a humidifier 5000, an air circuit 4170, and a patient interface 3000.

## 5.3 PATIENT INTERFACE

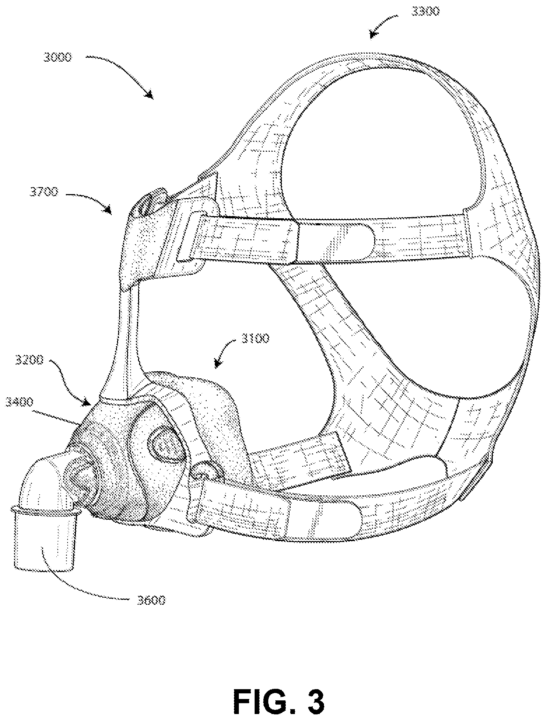

An example non-invasive patient interface 3000 is shown in Figure 3 and comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to facilitate the supply of air at positive pressure to the airways.

The patient interface 3000 in accordance with one form of the present technology is constructed and arranged to be able to provide a supply of air at a positive pressure, such as of at least 4 $cmH_2O$, or at least 10$cmH_2O$, or at least 20 $cmH_2O$, or at least 25 $cmH_2O$ with respect to ambient.

### 5.3.1 Seal-forming structure

In one form of the present technology, a seal-forming structure 3100 provides a target seal-forming surface region, and may additionally provide a cushioning function. The target seal-forming region is a region on the seal-forming structure 3100 where sealing may occur. The region where sealing actually occurs- the actual sealing surface-may change within a given treatment session, from day to day, and from patient to patient, depending on a range of factors including for example, where the patient interface was placed on the face, tension in the positioning and stabilising structure and the shape of a patient's face.

### 5.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material. An acoustic generator 8500 may be formed as a part of, or through a shell of, the plenum chamber 3200.

### 5.3.3 Positioning and stabilising structure

The seal-forming structure 3100 of the patient interface 3000 of the present technology may be held in sealing position in use by the positioning and stabilising structure 3300.

### 5.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use. One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 5.3.5 Connection port

Connection port 3600 allows for connection to the air circuit 4170, and may optionally include an integrated acoustic generator 8500.

## 5.4 RPT DEVICE

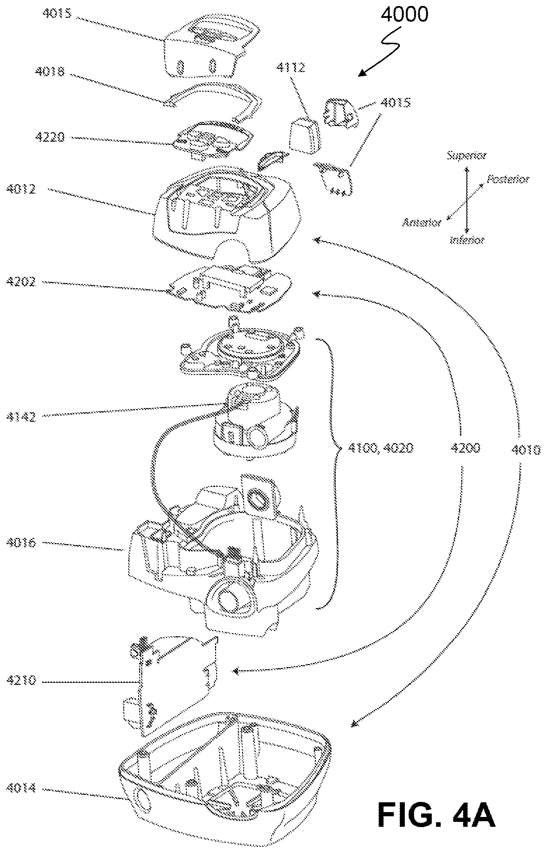

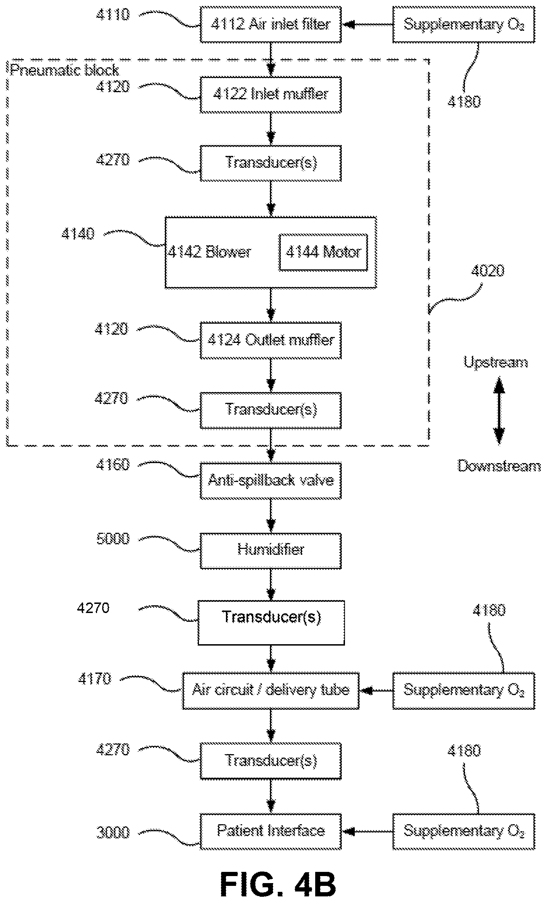

A respiratory pressure therapy (RPT) device 4000 in accordance with one aspect of the present technology is shown in exploded view in Figure 4A and comprises mechanical, pneumatic, and/or electrical components and is configured to execute one or more algorithms 4300. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 4 $cmH_2O$, or at least 10$cmH_2O$, or at least 20 $cmH_2O$, or at least 25 $cmH_2O$.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more airpath items, e.g., an inlet air filter 4112, an inlet muffler 4122, a pressure generator 4140 capable of supplying air at positive pressure (e.g., a blower 4142), an outlet muffler 4124 and one or more transducers 4270, such as pressure sensors and flow rate sensors.

One or more of the airpath items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices 4220, a central controller 4230, a therapy device controller 4240, a pressure generator 4140, one or more protection circuits 4250, memory 4260, transducers 4270, data communication interface 4280 and one or more output devices 4290. Electrical components 4200 may be mounted on a single Printed Circuit Board Assembly (PCBA) 4202. In an alternative form, the RPT device 4000 may include more than one PCBA 4202.

### 5.4.1 RPT device mechanical & pneumatic components

An RPT device may comprise one or more of the following components in an integral unit. In an alternative form, one or more of the following components may be located as respective separate units.

### 5.4.1.1 Pressure generator

In one form of the present technology, a pressure generator 4140 for producing a downstream air flow such as a flow, or a supply, of air at positive pressure is a controllable blower 4142. The blower may be capable of delivering a supply of air, for example at a rate of up to about 120 litres/minute, at a positive pressure in a range from about 4 $cmH_2O$ to about 20 $cmH_2O$, or in other forms up to about 30 $cmH_2O$. The blower may be as described in any one of the following patents or patent applications: U.S. Patent No. 7,866,944; U.S. Patent No. 8,638,014; U.S. Patent No. 8,636,479; and PCT Patent Application Publication No. WO 2013/020167.

The pressure generator 4140 is under the control of the therapy device controller 4240.

In other forms, a pressure generator 4140 may be a piston-driven pump, a pressure regulator connected to a high pressure source (e.g. compressed air reservoir), or a bellows.

### 5.4.1.2 Memory

In accordance with one form of the present technology the RPT device 4000 includes memory 4260, e.g., non-volatile memory. In some forms, memory 4260 may include battery powered static RAM. In some forms, memory 4260 may include volatile RAM.

Memory 4260 may be located on the PCBA 4202. Memory 4260 may be in the form of EEPROM, or NAND flash.

Additionally or alternatively, RPT device 4000 includes a removable form of memory 4260, for example a memory card made in accordance with the Secure Digital (SD) standard.

In one form of the present technology, the memory 4260 acts as a non-transitory computer readable storage medium on which is stored computer program instructions or processor control instructions expressing the one or more methodologies described herein, such as the one or more algorithms 4300.

*5.4.1.3 Data communication systems*

In one form of the present technology, a data communication interface 4280 is provided, and is connected to the central controller 4230. Data communication interface 4280 may be connectable to a remote external communication network 4282 and/or a local external communication network 4284. The remote external communication network 4282 may be connectable to a remote external device 4286. The local external communication network 4284 may be connectable to a local external device 4288.

In one form, data communication interface 4280 is part of the central controller 4230. In another form, data communication interface 4280 is separate from the central controller 4230, and may comprise an integrated circuit or a processor.

In one form, remote external communication network 4282 is the Internet. The data communication interface 4280 may use wired communication (e.g. via Ethernet, or optical fibre) or a wireless protocol (e.g. CDMA, GSM, LTE) to connect to the Internet.

In one form, local external communication network 4284 utilises one or more communication standards, such as Bluetooth, or a consumer infrared protocol.

In one form, remote external device 4286 is one or more computers, for example a cluster of networked computers. In one form, remote external device 4286 may be virtual computers, rather than physical computers. In either case, such a remote external device 4286 may be accessible to an appropriately authorised person such as a clinician.

The local external device 4288 may be a personal computer, mobile computing device such as a smartphone or tablet device, or a remote control.

*5.4.2 RPT device algorithms*

As mentioned above, in some forms of the present technology, the central controller 4230 may be configured to implement one or more algorithms 4300 expressed as computer programs stored in a non-transitory computer readable storage medium, such as memory 4260. The algorithms 4300 are generally grouped into groups referred to as modules.

**5.5 HUMIDIFIER**

*5.5.1 Humidifier overview*

In one form of the present technology the RT system comprises a humidifier 5000 between the RPT device 4000 and the air circuit 4170 (as shown in Fig. 4A) to change the absolute humidity of air for delivery to a patient relative to that of ambient air. Typically, the humidifier 5000 is used to increase the absolute humidity and increase the temperature of the flow of air (relative to ambient air) before delivery to the patient's airways.

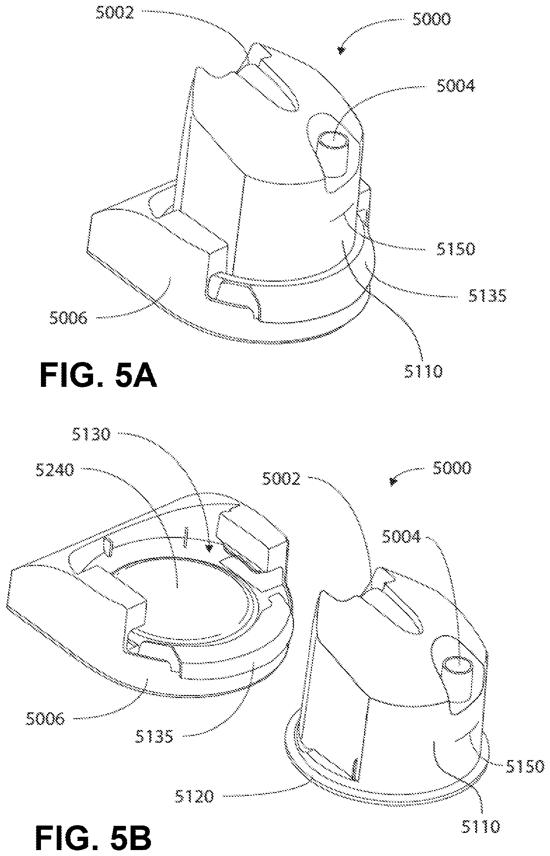

The humidifier 5000 (e.g. as shown in Fig. 5A) may comprise a humidifier reservoir 5110, a humidifier inlet 5002 to receive a flow of air, and a humidifier outlet 5004 to deliver a humidified flow of air. In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

*5.5.2 Humidifier components*

*5.5.2.1 Water reservoir*

According to one arrangement, the humidifier 5000 may comprise a water reservoir 5110 configured to hold, or retain, a volume of liquid (e.g. water) to be evaporated for humidification of the flow of air. The water reservoir 5110 may be configured to hold a predetermined maximum volume of water in order to provide adequate humidification for at least the duration of a respiratory therapy session, such as one evening of sleep. Typically, the reservoir 5110 is configured to hold several hundred millilitres of water, e.g. 300 millilitres (ml), 325 ml, 350 ml or 400 ml. In other forms, the humidifier 5000 may be configured to receive a supply of water from an external water source such as a building's water supply system.

According to one aspect, the water reservoir 5110 is configured to add humidity to a flow of air from the RPT device 4000 as the flow of air travels therethrough. In one form, the water reservoir 5110 may be configured to encourage the flow of air to travel in a tortuous path through the reservoir 5110 while in contact with the volume of water therein.

According to one form, the reservoir 5110 may be removable from the humidifier 5000, for example in a lateral direction as shown in Fig. 5A and Fig. 5B.

The reservoir 5110 may also be configured to discourage egress of liquid therefrom, such as when the reservoir 5110 is displaced and/or rotated from its normal, working orientation, such as through any apertures and/or in between its subcomponents. As the flow of air to be humidified by the humidifier 5000 is typically pressurised, the reservoir 5110 may also be configured to prevent losses in pneumatic pressure through leak and/or flow impedance.

### 5.5.2.2 Conductive portion

According to one arrangement, the reservoir 5110 comprises a conductive portion 5120 configured to allow efficient transfer of heat from the heating element 5240 to the volume of liquid in the reservoir 5110. In one form, the conductive portion 5120 may be arranged as a plate, although other shapes may also be suitable. All or a part of the conductive portion 5120 may be made of a thermally conductive material such as aluminium (e.g. approximately 2 mm thick, such as 1 mm, 1.5 mm, 2.5 mm or 3 mm), another heat conducting metal or some plastics. In some cases, suitable heat conductivity may be achieved with less conductive materials of suitable geometry.

### 5.5.2.3 Humidifier reservoir dock

In one form, the humidifier 5000 may comprise a humidifier reservoir dock 5130 (as shown in Fig. 5B) configured to receive the humidifier reservoir 5110. In some arrangements, the humidifier reservoir dock 5130 may comprise a locking feature such as a locking lever 5135 configured to retain the reservoir 5110 in the humidifier reservoir dock 5130.

### 5.5.2.4 Water level indicator

The humidifier reservoir 5110 may comprise a water level indicator 5150 as shown in Fig. 5A-5B. In some forms, the water level indicator 5150 may provide one or more indications to a user such as the patient 1000 or a care giver regarding a quantity of the volume of water in the humidifier reservoir 5110. The one or more indications provided by the water level indicator 5150 may include an indication of a maximum, predetermined volume of water, any portions thereof, such as 25%, 50% or 75% or volumes such as 200 ml, 300 ml or 400ml.

### 5.5.2.5 Humidifier transducer(s)

The humidifier 5000 may comprise one or more humidifier transducers (sensors) 5210 instead of, or in addition to, transducers 4270 described above. Humidifier transducers 5210 may include one or more of an air pressure sensor 5212, an air flow rate transducer 5214, a temperature sensor 5216, or a humidity sensor 5218. A humidifier transducer 5210 may produce one or more output signals which may be communicated to a controller such as the central controller 4230 and/or the humidifier controller 5250. In some forms, a humidifier transducer may be located externally to the humidifier 5000 (such as in the air circuit 4170) while communicating the output signal to the controller 5250.

### 5.6 AIR CIRCUIT

An air circuit 4170 in accordance with an aspect of the present technology is a conduit or a tube constructed and arranged to allow, in use, a pressurised flow of air to travel between two components such as the humidifier 5000 and the patient interface 3000.
In particular, the air circuit 4170 may be in fluid communication with the outlet 5004 of the humidifier 5000 and the plenum chamber 3200 of the patient interface 3000.

### 5.7 TRANSDUCER(S)

An RT system may comprise one or more transducers (sensors) 4270 configured to measure one or more of any number of parameters in relation to an RT system, its patient, and/or its environment. A transducer may be configured to produce an output signal representative of the one or more parameters that the transducer is configured to measure.
The output signal may be one or more of an electrical signal, a magnetic signal, a mechanical signal, a visual signal, an optical signal, a sound signal, or any number of others which are known in the art.
A transducer may be integrated with another component of an RT system, where one exemplary arrangement would be the transducer being internal of an RPT device. A transducer may be substantially a 'standalone' component of an RT system, an exemplary arrangement of which would be the transducer being external to the RPT device.
A transducer may be configured to communicate its output signal to one or more components of an RT system, such as an RPT device, a local external device, or a remote external device. External transducers may be for example located on a patient interface, or in an external computing device, such as a smartphone. External transducers may be located for example on or form part of the air circuit, e.g., the patient interface.
The one or more transducers 4270 may be constructed and arranged to generate signals representing properties of air such as a flow rate, a pressure or a temperature. The air may be a flow of air from the RPT device to a patient, a flow of air from the patient to the atmosphere, ambient air or any others. The signals may be representative of properties of the flow of air at a particular point, such as the flow of air in the pneumatic path between the RPT device and the patient. In one form of the present technology, one or more transducers 4270 are located in a pneumatic path of the RPT device, such as downstream of the humidifier 5000.

*5. 7.1 Pressure sensor*

In accordance with one aspect of the present technology, the one or more transducers 4270 comprises a pressure sensor located in fluid communication with the pneumatic path. An example of a suitable pressure sensor is a transducer from the HONEYWELL ASDX series. An alternative suitable pressure sensor is a transducer from the NPA Series from GENERAL ELECTRIC. In one implementation, the pressure sensor is located in the air circuit 4170 adjacent the outlet 5004 of the humidifier 5000.

The pressure sensor (microphone) 4270 is configured to generate a sound signal representing the variation of pressure within the air circuit 4170. The sound signal from the microphone 4270 may be received by the central controller 4230 for acoustic processing and analysis as configured by one or more of the algorithms 4300 described below. The microphone 4270 may be directly exposed to the airpath for greater sensitivity to sound, or may be encapsulated behind a thin layer of flexible membrane material. This membrane may function to protect the microphone 4270 from heat and/or humidity.

## 5.8 ACOUSTIC ANALYSIS

According to one or more aspects of the present technology, acoustic analysis may be used to determine one or more parameters in relation to respiratory disorders, or systems for treatment of respiratory disorders.

Acoustic analysis according to aspects of the present technology may have one or more advantages over the prior art, such as reducing cost of care, delivering higher quality of therapy, improving ease of use of a therapy system, reducing waste, and providing digital connectivity at a low cost.

As will be clear in the context of the remainder of the document, the terms 'acoustic', 'sound', or 'noise' in the present document are generally intended to include airborne vibrations, regardless of whether they may be audible or inaudible. As such, the terms 'acoustic', 'sound', or 'noise' in the present document are intended to include airborne vibrations in the ultrasonic, or subsonic ranges, unless specifically stated otherwise.

Some implementations of the disclosed acoustic analysis technologies may implement cepstrum analysis. A cepstrum may be considered the inverse Fourier Transform of the log spectrum of the forward Fourier Transform of the decibel spectrum, etc. The operation essentially can convert a convolution of an impulse response function (IRF) and a sound source into an addition operation so that the sound source may then be more easily accounted for or removed so as to isolate data of the IRF for analysis. Techniques of cepstrum analysis are described in detail in a scientific paper entitled "The Cepstrum: A Guide to Processing" (Childers et al, Proceedings of the IEEE, Vol. 65, No. 10, Oct 1977) and Randall RB, Frequency Analysis, Copenhagen: Bruel & Kjaer, p. 344 (1977, revised ed. 1987). The application of cepstrum analysis to respiratory therapy system component identification is described in detail in PCT publication no. WO2010/091462, titled "Acoustic Detection for Respiratory Treatment Apparatus".

Cepstrum analysis may be understood in terms of the property of convolution. The convolution of *f* and g can be written as *f*g. This operation may be the integral of the product of the two functions (*f* and g) after one is reversed and shifted. As such, it is a type of integral transform as follows:

$$(f * g)(t) = \int_{-\infty}^{\infty} f(\tau)g(t-\tau)d\tau \qquad (1)$$

While the symbol *t* is used above, it need not represent the time domain. But in that context, the convolution formula can be described as a weighted average of the function *f*($\tau$) at the moment *t* where the weighting is given by *g*(-$\tau$) simply shifted by amount *t*. As *t* changes, the weighting function emphasizes different parts of the input function.

A mathematical model that can relate output to the input for a time-invariant linear acoustic system, such as the pneumatic path of a respiratory therapy system, can be based on convolution. The sound signal generated by a microphone 4270 in the air circuit 4170 may be considered as an input sound signal "convolved" with the system Impulse Response Function (IRF) as a function of time (t).

$$y(t) = s_1(t) * h_1(t) \qquad (2)$$

where $y(t)$ is the output sound signal generated by the microphone 4270; $s_1(t)$ is the input sound signal such as sound generated in or by a pressure generator 4140 of a respiratory therapy device 4000, and $h_1(t)$ is the system IRF from the sound source to the microphone 4270. The system IRF $h_1(t)$ may be thought of as the system response to a unit impulse input.

Conversion of Equation (2) into the frequency domain by means of the Fourier Transform of the sound signal $y(t)$ (e.g., a discrete Fourier Transform ("DFT") or a fast Fourier transform ("FFT")) and considering the Convolution Theorem, the following equation is produced:

$$Y(f) = S_1(f) H_1(f) \qquad (3)$$

where $Y(f)$ is the Fourier Transform (spectrum) of $y(t)$; $S_1(f)$ is the Fourier Transform of $s_1(t)$; and $H_1(f)$ is the Fourier Transform of $h_1(t)$. In other words, a convolution in the time domain becomes a multiplication in the frequency domain.

A logarithm operation may be applied to Equation (3) so that the multiplication is converted into an addition:

$$Log\{Y(f)\} = Log\{S_1(f)\} + Log\{H_1(f)\} \qquad (4)$$

Equation (4) may then be converted back into the time domain, by an Inverse Fourier Transform (IFT) (e.g., an inverse DFT or inverse FFT), which results in a complex-valued "Cepstrum" - the inverse Fourier Transform of the logarithm of the spectrum $Y(f)$:

$$
\begin{aligned}
\tilde{Y}(\tau) &= IFT[Log\{Y(f)\}] \qquad (5)\\
&= IFT[Log\{S_1(f)\}] + IFT[Log\{H_1(f)\}]\\
&= \widetilde{S_1}(\tau) + \widetilde{H_1}(\tau)
\end{aligned}
$$

The abscissa $\tau$ is a real-valued variable known as quefrency, with units measured in seconds. Effects that are convolutive in the time domain thus become additive in the logarithm of the spectrum, and remain so in the cepstrum or quefrency domain. In particular, the output cepstrum $\tilde{Y}(\tau)$ consists of two additive components: the cepstrum $\tilde{S}_1(\tau)$ of the input signal $s_1(t)$, and the cepstrum $\widetilde{H_1}(\tau)$ of the system IRF $h_1(t)$.

Consideration of the data from a cepstrum analysis, such as examining the data values in the quefrency domain, may provide information about the RT system. For example, by comparing cepstrum data of a system from a prior or known baseline of cepstrum data for the system, the comparison, such as a difference, can be used to recognize differences or similarities in the system that may then be used to implement automated control for varying functions or purposes.

*5.8.1 Component identification*

As previously mentioned, a respiratory therapy system typically may include an RPT device, a humidifier, an air delivery conduit, and a patient interface. A variety of different forms of patient interface may be used with a given RPT device, for example a nasal pillows, nasal prongs, nasal mask, nose & mouth (oronasal) mask, or full face mask. Furthermore, different forms of air delivery conduit may be used. Moreover, even for a common form, such as a nasal mask, there may be differences in design specifications, such as size and/or shape, for different models of the form of patient interface (e.g., different nasal masks). In order to provide improved control of therapy delivered to the patient interface, it may be advantageous to measure or estimate treatment parameters such as pressure in the patient interface, and vent flow. In systems using estimation of treatment pressures, knowledge of the type of component being used by a patient can enhance the accuracy of treatment pressure estimation, and therefore the efficacy of therapy. To obtain that knowledge, some RPT devices include a menu system that allows the patient to select the type of system components, including the patient interface, being used, e.g., brand, form, model, etc. Once the types of the components are entered by the patient, the RPT device controller can select appropriate operating parameters of the RPT device that best coordinate with the selected components.

The present technology may provide improvements to known apparatus to facilitate the coordination between the RPT device and the peripheral components of the RT system based on acoustic analysis to identify components of the RT system. In the present specification, "identification" of a component means identification of the type of that component so as to distinguish the component from other, different (e.g., pneumatically different), component types that might be used with the RT system. Such identification may then permit access to data associated with the identification, such as pneumatic characteristics, that may then be applied by a controller of the RT device in its pneumatic control (e.g., therapy control). In what follows, "mask" is used synonymously with "patient interface" for brevity, even though there exist patient interfaces that are not usually described as "masks".

A first embodiment of the present technology comprises a device, an apparatus, and/or a method for identifying component(s) in a respiratory therapy system. The components may be masks and conduits. This embodiment may identify the length of the conduit in use, as well as the mask connected to the conduit. The technology may identify the mask and conduit regardless of whether a patient is wearing the mask at the time of identification.

The present technology may implement analysis of a sound signal generated by a microphone 4270 located as described above.

The present technology includes an analysis method that enables the separation of the acoustic mask reflections from the other system noises and responses, including but not limited to blower sound. This makes it possible to identify differences between different masks' acoustic reflections (usually dictated by mask shapes, configurations and materials) and may permit the identification of different masks such as without requiring user or patient intervention.

An example method of identifying the mask is to sample the output sound signal $y(f)$ generated by the microphone 4270 at a desired sampling rate, such as at least the Nyquist rate, for example 20 kHz. The cepstrum $\tilde{Y}(\tau)$ may be computed from the sampled output signal. A reflection component of the cepstrum may then be separated from the input signal component of the cepstrum. The reflection component of the cepstrum comprises the acoustic reflection from the mask of the input sound signal, and is therefore referred to as the "acoustic signature" of the mask, or the "mask signature". The acoustic signature may then be compared with a predefined or predetermined database, such as any suitable type of data storage structure, of previously measured acoustic signatures obtained from systems containing known masks. Optionally, some criteria may be set to determine appropriate similarity. In one example embodiment, the comparisons may be completed based on the single largest data peak in the cross-correlation between the measured and stored acoustic signatures. However, this approach may be improved by comparisons over several data peaks or alternatively, wherein the comparisons are completed on extracted unique sets of cepstrum features.

Alternatively, the same method may be also used to determine the conduit length, by finding the delay between a sound being received from the RPT device and its reflection from the mask being received; the delay may be proportional to the length of the tube. Additionally, changes in tubing diameter may increase or decrease the amplitude of the reflected signals and therefore may also be identifiable. Such an assessment may be made by comparison of current reflection data with prior reflection data. The diameter change may be considered as a proportion of the change in amplitude from the reflected signals (i.e., reflection data).

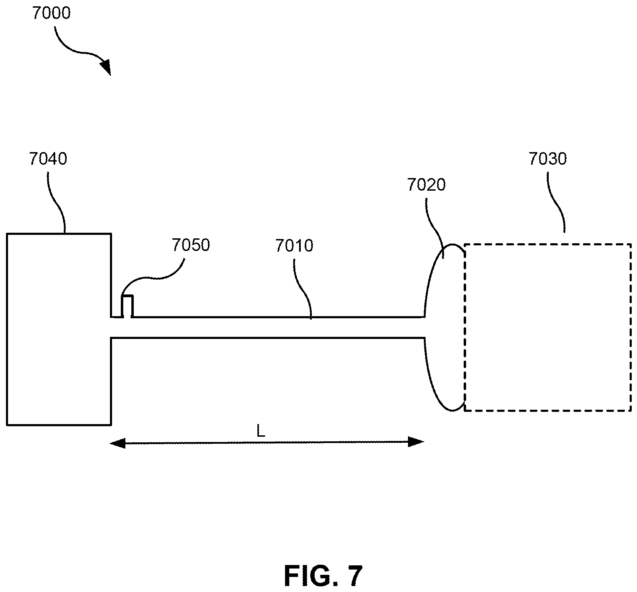

Fig. 7 is a schematic view of an RT system 7000 according to one aspect of the present technology. In this example embodiment as shown in Fig. 7, the conduit 7010 (of length $L$) effectively acts as an acoustic waveguide for sound produced by the RPT device 7040, such as including sound from a speaker, or alternatively only noise of the operation of the blower (e.g., motor and/or impeller). In this example embodiment the input signal is sound emitted by the RPT device 7040 (i.e., without sound from a speaker). The input signal (e.g. an impulse) enters the microphone 7050 positioned at one end of the conduit 7010, travels along the airpath in conduit 7010 to mask 7020 and is reflected back along the conduit 7010 by features in the airpath (which includes the conduit and the mask) to enter the microphone 7050 once more. The system IRF (the output signal produced by an input impulse) therefore contains an input signal component and a reflection component. A key feature of the RT system 7000 is the time taken by sound to travel from one end of the airpath to the opposite end. This interval is manifested in the system IRF because the microphone 7050 receives the input signal coming from the RPT device 7040, and then some time later receives the input signal filtered by the conduit 7010 and reflected and filtered by the mask 7020 (and potentially any other system 7030 attached to the mask, e.g. a human respiratory system when the mask 7020 is seated on a patient). This means that the component of the system IRF associated with the reflection from the mask end of the conduit 7010 (the reflection component) is delayed in relation to the component of the system IRF associated with the input signal (the input signal component), which arrives at the microphone 7050 after a relatively brief delay. (For practical purposes, this brief delay may be ignored and zero time approximated to be when the microphone 7050 first responds to the input signal.) The delay is equal to $2L/c$ (wherein $L$ is the length of the conduit, and $c$ is the speed of sound in the conduit).

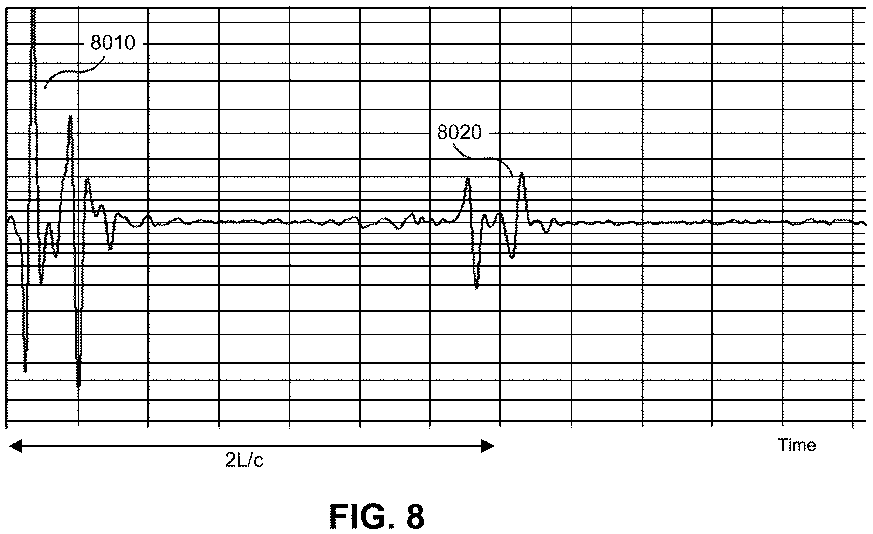

Another feature of the system 7000 is that because the airpath is loss-prone, provided the conduit is long enough, the input signal component of the system IRF decays to a negligible amount by the time the reflection component of the system IRF has begun. If this is the case, then the input signal component may be separated from the reflection component of the system IRF. As an example, Fig. 8 shows an example of one such system IRF from an example therapy system in which the input signal may originate from the blower 4142 of the RPT device. Alternatively, the input signal may include sound originated from a speaker at the device end of the airpath (with or without sound generated by the RPT device 7040). Fig. 8 shows that the reflection component 8020 of the system IRF appears delayed from the input signal component 8010 in the system IRF, with a delay equal to $2L/c$.

The cepstrum $\tilde{H}_1(\tau)$ of the system IRF associated with equations (2), (4), and (5) previously described has generally the same the properties as the system IRF $h_1(t)$. That is, the cepstrum $\tilde{H}_1(\tau)$ comprises a reflection component concentrated around a quefrency of $2L/c$ as well as an input signal component concentrated around quefrency zero. In the present technology, the cepstrum analysis is configured to separate the reflection component of the output cepstrum $\tilde{Y}(\tau)$ from other system artifacts (including but not limited to the input signal component $\widetilde{S}_1(\tau)$ ), e.g., by examination of the position and amplitude of the output cepstrum data.

This separation may be accomplished if the input signal $s_1(t)$ is either transient (e.g. an impulse), or stationary random, which means the input signal component $\widetilde{S}_1(\tau)$ of the cepstrum will be concentrated around a quefrency of zero. For example, the input signal may be the sound produced by an RPT device running at a constant speed during the time period of the microphone's measurement. This sound may be described as "cyclostationary". That is, it is stationary random, and periodic in its statistics. This means that the input signal component and the reflection component of the system IRF may be "smeared" across all measured times of the output signal $y(t)$ because at any point in time, the output signal $y(t)$ is a function of all previous values of the input signal and system IRF (see equation (2)). However, the cepstrum analysis described above may be implemented to separate the reflection component of the output cepstrum $\tilde{Y}(\tau)$ from this convolutive mixture.

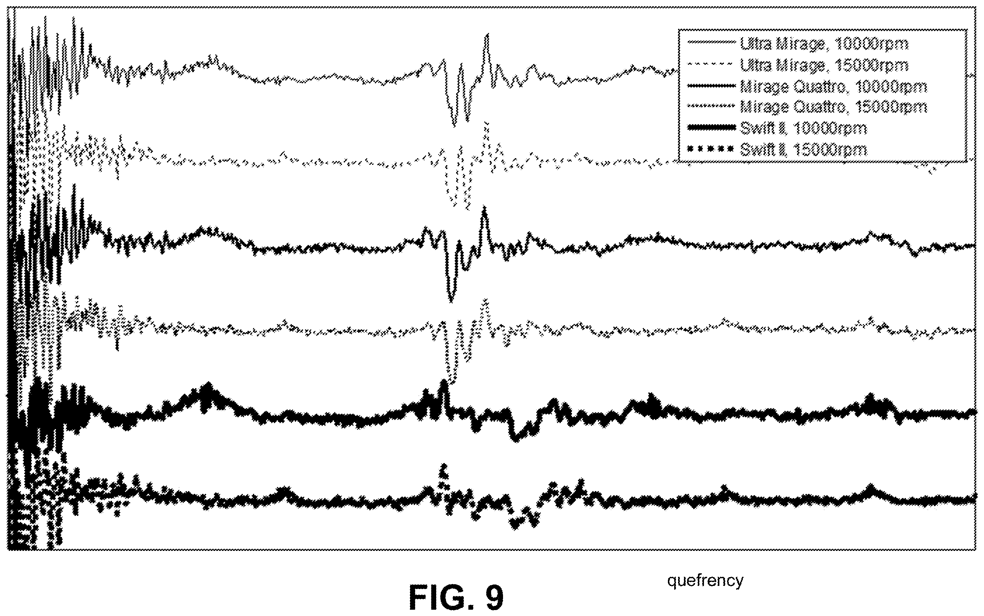

Fig. 9 depicts the real part of various example cepstra from measurements of a therapy system, such as the system of Fig. 7, implemented with three different masks, with the input signal being the sound produced by the RPT device blower. Each mask in this example was tested at two different operational speeds of the blower, namely 10 krpm and 15 krpm. Although these speeds were used in the examples, the methodology may be implemented with other blower speeds particularly if the resulting sound is detectable by the microphone 7050.

In Fig. 9, the reflection component can clearly be seen in all six cepstra, beginning at around a quefrency of twelve milliseconds (12 ms). This position is as expected ($2L$ / c) since in the example therapy system, a two-meter conduit was used and the speed of sound is 343m/s. In Fig. 9, the graph illustrates cepstra from masks in the following order from top to bottom:

- ResMed Ultra Mirage™ at 10 krpm;

- ResMed Ultra Mirage™, at 15 krpm;

- ResMed Mirage Quattro™ at 10 krpm;

- ResMed Mirage Quattro™ at 15 krpm;

- ResMed Swift II™ at 10 krpm; and

- ResMed Swift II™ at 15 krpm.

By increasing the conduit length, an appreciable increase in the delay of arrival of the reflection from the mask may also be achieved. The increase in delay, when compared to Fig. 9, is in accordance with the aforementioned calculations generating the approximations of the conduit length.

In accordance with the present technology, data associated with the reflection component, such as that centrally illustrated in the cepstra of Fig. 9, may then be compared with similar data from a set of previously identified mask reflection components or set of predetermined acoustic signatures such as that contained in a memory or database of mask reflection components. Such a set may contain data from one or more of such previously identified mask reflection components.

For example, the reflection component of a mask being tested (the "mask signature") may be separated from the cepstrum of the output signal generated by the microphone. This mask signature may be compared to that of previous or predetermined mask signature(s) of known masks stored as a data template of the apparatus, such as to confirm identification of a known mask. One way of doing this is to calculate the cross-correlation between the mask signature of the mask being tested and previously stored mask signatures for all known masks or data templates. There is a high probability that the cross-correlation with the highest peak corresponds to the mask being tested, and the location of the peak should be proportional to the length of the conduit.

However, more points of correlation may also increase the accuracy of the identification steps of the present technology. Thus, additional data points may be utilized. Optionally, a least squares algorithm with the test data and known data sets may be implemented in the component identification. Still further, in some embodiments additional feature extraction and recognition techniques may be utilized, which may be based on artificial intelligence / machine learning structures and strategies such as a neural network or a support vector machine. Other sources of information may also be included as inputs to such structures and strategies to improve the accuracy of component identification. Examples are patient characteristics, therapy data, historical information such as previously identified components, geographic location and associated market data such as sales figures for various components.

One complicating factor in acoustic component identification is acoustic "back-reflections" from the device end of the conduit 7010. These back-reflections occur from the device end of the conduit 7010 after the sound reflected from the mask 7020 has travelled back along the conduit 7010, as a result of the change in acoustic impedance between the conduit 7010 and the interior cavity of the RPT device / humidifier 7040 to which the conduit 7010 is connected. Such back-reflections may have a muddying effect on the acoustic signature of the mask. For example, when the dimensions of the component being identified are of a similar physical scale to the distance between the microphone 7050 and any discontinuity in the cross-sectional area inside the RPT device / humidifier 7040, the reflection from the component may be superimposed in the output signal on the response of the back-reflection.

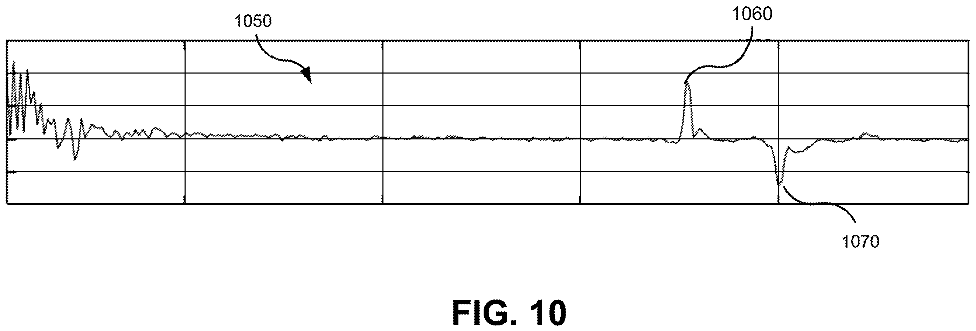

Fig. 10 contains a graph illustrating this effect. The trace 1050 is an output cepstrum containing a clear peak 1060 at the quefrency associated with the sound travelling from the microphone to the component to be identified and being reflected from that component back to the microphone. The cepstrum trace 1050 also contains a clear trough 1070 at the quefrency that is collectively associated with (a) the sound travelling from the microphone to the component, (b) reflection from the component back down the conduit to the blower device end of the conduit, and (c) back-reflection from the blower device end to the microphone.

In some cases, component identification might be made more accurate by characterizing the back-reflections and deconvolving them from the reflection component. Alternatively, component identification might be made more accurate if back-reflection could be reduced or minimised by design. The latter case is complicated by the need for maintaining an open passage from the blower end to the patient interface so that there may be provision of therapy pressure into the waveguide that is formed by the therapy conduits.

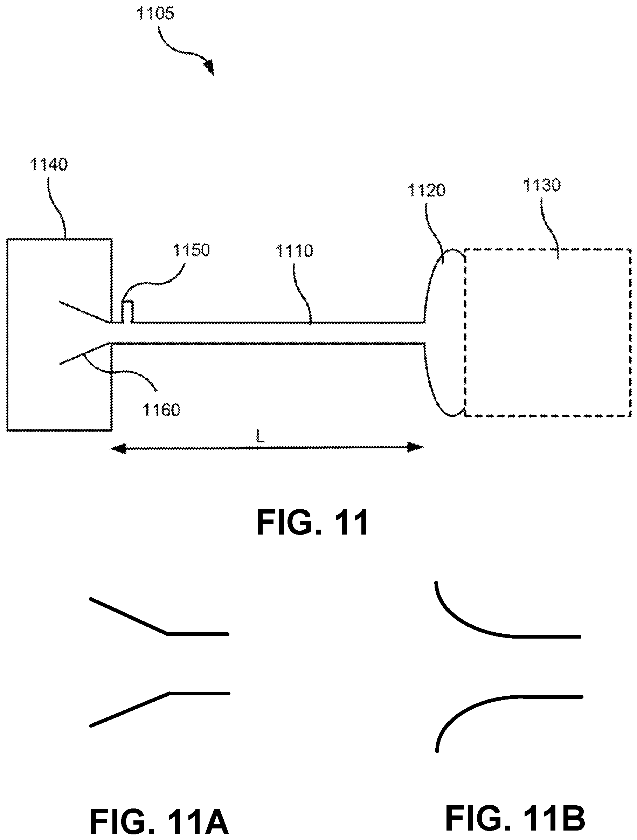

In one such implementation 1105, illustrated in Fig. 11, the end of the conduit 1110 nearest the microphone 1150, distal from the patient respiratory airways 1130, comprises a dampening structure 1160 configured to reduce back-reflection. The structure of such a dampening structure may be generally open such that it has a passage that permits airflow (for therapy) and sound through it and as such may be considered a pass-through (acoustic) dampening conduit. Such a pass-through dampening conduit may be a transitional conduit passage that has an internal surface that defines a cross-section of the passage through the structure where that cross-section expands, due to the internal surface profile, such as in a linear manner, along an acoustic or airflow path of the conduit passage. As illustrated, the structure 1160 may be located along the conduit between the microphone and the blower, such that the microphone is between the structure and the patient interface along the conduit. The example of the structure 1160 of Fig. 11 is illustrated as a horn that extends from the device end of the conduit 1110, where its diameter may be the same of that of the conduit 1110, into an interior cavity of the RPT device / humidifier 1140 with a gradually increasing cross-section or transverse dimension (e.g., diameter) such as in a direction away from the component to be identified. In this regard, the cross-section of the structure 1160, due to its internal surface profile, expands as the cross-section is further from the patient interface end of the patient circuit. Because acoustic impedance of an acoustic waveguide is related to the internal transverse dimension (e.g. diameter) of the waveguide, the horn structure 1160, unlike an absorbing material, minimises back-reflection by gradualising the change in acoustic impedance between the conduit 1110 and the cavity of the RPT device / humidifier 1140. The horn structure 1160 may be of conical profile as illustrated in Figs. 11 and 11A, or the profile of the horn may curve in the manner of the bell of a brass instrument such as in the example of Fig. 11B. The effect of the structure 1160 is to reduce the back-reflection component in the acoustic signature of a system component 1120 and therefore increase the identifiability of the system component.

The cepstrum may be computed over a finite time window of the sampled output signal $y(t)$. A longer window may produce a cleaner separation between the acoustic signature and the input signal component. However, since the acoustic characteristics of the airpath may vary over time due to such factors as the change in pressure and / or flow rate and / or humidity over the breathing cycle, such as if evaluated while in use by a patient, the window should not be made so long that significant variation of the airpath characteristics over the window may be expected. In one example, the window is of duration 200 ms.

Subtracting a low-pass filtered version of the log spectrum $Log\{Y(f)\}$ from itself, e.g. subtracting a moving average of the log spectrum $Log\{Y(f)\}$ from the log spectrum $Log\{Y(f)\}$, before performing the IFT in Equation (5) to compute the cepstrum $\tilde{Y}(\tau)$, may flatten the global shape of the log spectrum and reduce the sensitivity of the separation of the acoustic signature to the randomness of the input signal $s_1(t)$. That is, such flattening will concentrate the input signal component $\tilde{S}_1(\tau)$ in the output cepstrum $\tilde{Y}(\tau)$ around the origin ($\tau = 0$) even if the input signal $s_1(t)$ is not particularly random in character. This concentration increases the separability of the input signal component $\tilde{S}_1(\tau)$ from the reflection component of the system IRF (the acoustic signature) in the output cepstrum $\tilde{Y}(\tau)$ for a given input signal. Care needs to be taken to set the filter such that the conduit resonant frequency is not removed in the flattening process. For example, the filter cut-off point should be low enough (or the window of the moving average long enough) that the conduit resonance is significantly removed by the filter, and thus preserved by the flattening process. In an alternative implementation, the log spectrum $Log\{Y(f)\}$ may be high-pass filtered before performing the IFT in Equation (5) to compute the cepstrum $\tilde{Y}(\tau)$.

Multiple output cepstra $\tilde{Y}(\tau)$ may be computed over multiple windows, and the acoustic signatures extracted from the respective cepstra may be combined together into a single, combined acoustic signature before comparing with the previously measured acoustic signatures (e.g., template data). In some implementations, the combination is an averaging of the multiple acoustic signatures. Such combination tends to reduce the effect of noise on the combined acoustic signature.

In order to minimise the varying effect of the breathing cycle on the acoustic characteristics of the airpath, and hence the variability of the acoustic signature between windows, the windows may be timed to coincide (synchronised) with a particular point in the breathing cycle, such as the peak of inspiratory flow rate or the pause at the end of expiration. In a similar manner, the window may be synchronised with a particular shaft rotation speed of the RT device in order to either reduce or highlight the influence of rotating machinery on the signature. In some embodiments, the acoustic analysis may include diagnosis or prognosis of machine conditions, such as bearing faults.

Even in such "breathing-synchronised" implementations, other sources of airpath variability between windows may affect the relative delay of each acoustic signature in the quefrency domain. Depending on how the acoustic signatures are combined, this may cause the combined acoustic signature to be smeared or blurred, affecting its distinctiveness.

In some implementations, therefore, a method of combination may be chosen that is robust to small variations in delay (relative shifts along the quefrency axis) between the multiple acoustic signatures. In one such implementation, the newly computed acoustic signatures are incorporated one by one into a combined acoustic signature so as to progressively construct a combined acoustic signature from each newly computed one. To achieve the incorporation, each newly computed acoustic signature may be compared with the combined acoustic signature to estimate its delay relative to the combined acoustic signature. The estimated relative delay may be compensated for before incorporating the newly computed acoustic signature into the combined acoustic signature by shifting the acoustic signature by its estimated delay. In one such implementation, the delay may be estimated by finding the location of a prominent feature in the acoustic signature such as its largest negative peak. In an alternative implementation, the delay may be estimated by correlating the acoustic signature with the combined acoustic signature and locating the correlation peak. The delay estimation and compensation of an acoustic signature may be referred to as alignment of the acoustic signature. In the case of combination by averaging, each shifted acoustic signature may then be averaged with the combined signature.

Other combination techniques may produce a combined acoustic signature that is robust to small variations in delay, e.g. a wavelet transform-based combination.

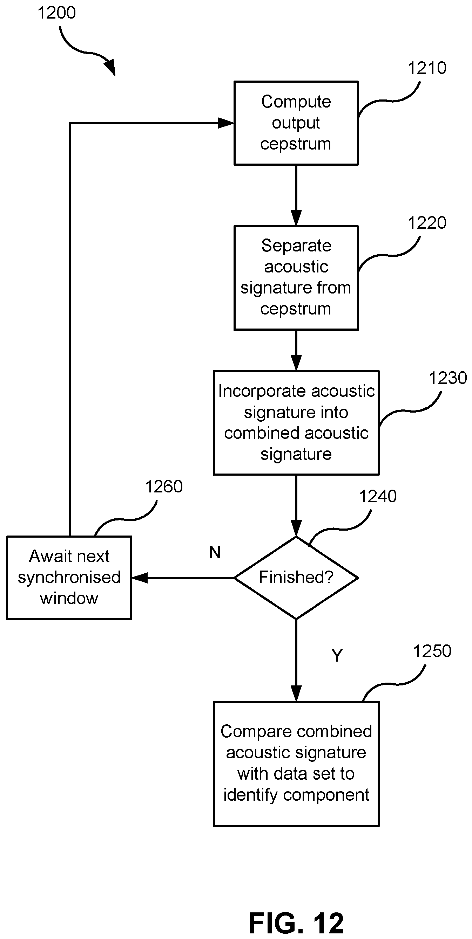

Fig. 12 is a flow chart illustrating a method 1200 of identifying a component of the airpath of a respiratory therapy system in accordance with one aspect of the present technology. The method 1200 may start at step 1210, which computes the output cepstrum $\tilde{Y}(\tau)$ from the output signal $y(t)$, such as during a breathing-synchronised window as described above in relation to equation (5). Step 1210 optionally flattens the log spectrum $Log\{Y(f)\}$ before computing the output cepstrum $\tilde{Y}(\tau)$ as described above.

Step 1220 follows, at which the reflection component (acoustic signature) is separated from the cepstrum computed at step 1210. At the next step 1230, the acoustic signature is incorporated into the combined acoustic signature in a manner robust to small shifts in the quefrency domain, as described above. (In a first pass through the loop, step 1230 simply designates the acoustic signature as the combined acoustic signature).

Step 1240 then checks whether sufficient acoustic signatures have been incorporated to make up the combined acoustic signature. If not ("N"), the method 11000 proceeds to step 1260, which awaits the next breathing-synchronised window before returning to step 1210 to compute a new cepstrum. If so ("Y"), step 1250 compares the combined acoustic signature with a predefined or predetermined signature data set such as from a database of previously measured acoustic signatures obtained from systems containing known components, to identify the current component. The method 1200 then concludes.

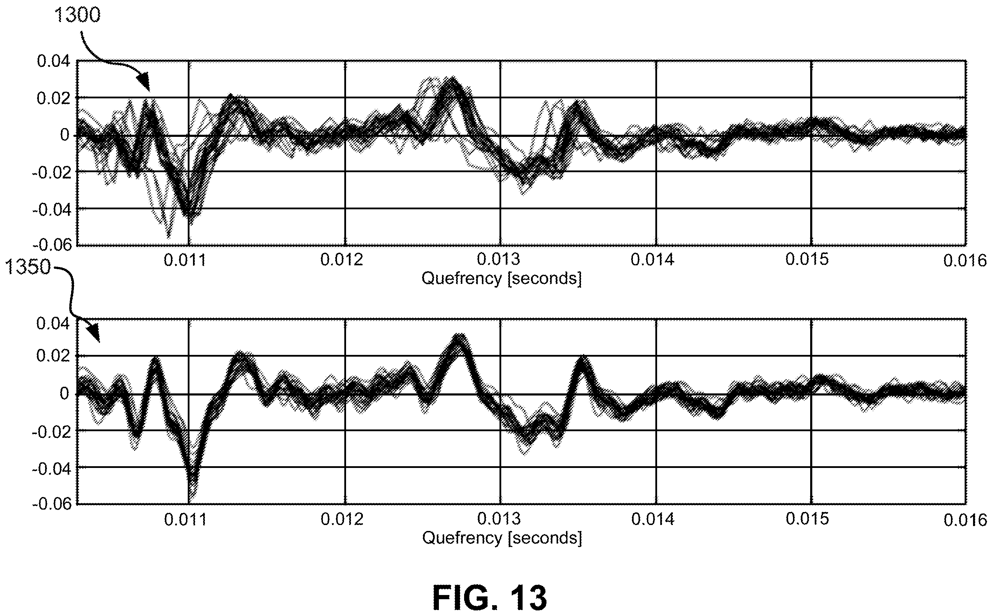

Fig. 13 contains two graphs. The top graph 1300 contains a set of unaligned acoustic signatures as tube length varies between windows. The bottom graph 1350 contains the same set of acoustic signatures after alignment according to the largest-negative-peak implementation described above. It may be seen that alignment produces a set of acoustic signatures that will result in a more sharply defined combined acoustic signature than will the set of unaligned acoustic signatures.

The signal processing analysis described in relation to Fig. 12 may be implemented by a controller or processor, such as with firmware, hardware and/or software as previously discussed. Such a controller may identify the mask and the conduit. This identification information or data relating to the identity of the patient interface, may then be relayed to a further controller, processor, system or computer or used by the controller. The information then may be utilized in adjusting therapy or other settings for the control of the RPT device in the delivery of respiratory therapy by the respiratory therapy system.

For example, the aforementioned technology may be implemented as part of a controller of a respiratory therapy system such as CPAP apparatus. Such an implementation may help to alleviate the need for users or clinicians of the CPAP apparatus to manually input or adjust the settings of the apparatus for use with particular masks. Thus, some embodiments of the technology may even permit users to change masks without user input or setup being required for the CPAP apparatus since such a system may automatically set the apparatus with settings adjusted in accordance with the automatically identified patient interface or mask configuration. In some cases, it may simplify setup by prompting the user with the identification from the aforementioned automated process so that the user may simply input confirmation of the identification, thereby avoiding or reducing the need for the user to scroll through many possible patient interface entries on a user interface of the RT device for the setup.

Additionally, in some embodiments, the information relating to the identity of a particular mask may be selectively transmitted to a manufacturer, doctor or clinician so that the information may be used to assist patients with troubleshooting. For example, such data could be transmitted by wireless communication protocols such as Bluetooth™ and/or WiFi™.

Additionally, in some embodiments, the information relating to the identity of a particular mask may be used to trigger action such as manual or automated deployment of personalised coaching or training content related to the particular mask, for example, a tutorial on making adjustments to the mask. Such material may be communicated to the user via the therapy device screen or a supporting mobile device application, or other means of communication such as email or SMS message.

Alternately, in some embodiments of the present technology, a controller may be configured to detect whether the patient is currently wearing the mask based on the nature of the acoustic reflection, for example, by comparing test cepstrum data to template cepstrum data recorded during patient use. Similarly, the present technology may be implemented to determine whether there is a technical problem with the mask including leaks and/or kinks in the system. This may also be detected by comparing current test cepstrum data to cepstrum data recorded while the mask was in good working order and properly seated on a patient's face (e.g., no leak).

In some embodiments, greater blower speeds than the speeds illustrated above may be implemented during component identification. For example, some conduits use materials with properties that may reduce noise. In such a system, the acoustic losses of the system may fluctuate. If the losses increase as detected by the measured signal (e.g., amplitude decrease), the decibels of the sound or noise source may be increased to overcome the effects of sound loss. This may be achieved by increasing the speed of the blower during a test measurement process. Additionally, other elements included in the airpath may increase the acoustic losses. These elements may include: humidifiers, noise baffles, and valves. Again, the loss attributable to these components may also be overcome by increasing the noise source level or amplitude. Typically, a suitable sound level for the input signal may be about 20 dBa or greater.

The frequency range of the microphone 4270 may be selected according to the geometric resolution required for the component identification. Resolving information about small dimensions will typically require high frequency content in the generated sound signal. A typical air circuit for respiratory therapy might exhibit tube resonances with a fundamental frequency of less than 100 Hz, but with higher harmonics appearing in the spectrum as integer multiples of the fundamental frequency up to more than 10 kHz. The frequency range of the microphone 4270 may be selected to be large enough to allow sensing of enough of the resonant harmonics that the periods associated with the harmonic spacing are present in the inverse Fourier Transform of the log spectrum. In one implementation, therefore, the microphone 4270 has an upper frequency limit of at least 10 kHz.

As previously mentioned, some embodiments may utilize a sound source such as a speaker to generate a sound impulse or white noise. This may be particularly useful for respiratory therapy systems with very quiet blowers that do not generate much noise. For example, when using a ResMed™ RPT device at speeds generally less than 6 krpm, the blower is very quiet. Under this condition, using only the sound of the blower as a sound source to produce the input signal might be insufficient for identification of some components. This may be overcome by including an additional sound source in the airpath. This may be activated during time periods of measurement such as when the mask is initially attached to the conduit. While an additional sound source might be a speaker, other sound emitters might be utilized. For example, a simple acoustic generator might be configured to vibrate in response to the flow of air from the RPT device such as a reed that may be selectively activated and deactivated (e.g., mechanically applied and removed from the airpath of the system). This may then serve to selectively create the sound impulse. Alternatively, an actuated valve of the RPT device may serve as the additional sound source.

Additionally, a sound source such as a speaker may be used to fill in gaps in the sound spectrum created by the blower. For example, a speaker may be used to produce a signal designed to have a particular spectrum such that the addition of the blower noise and the speaker sound produce a white spectrum. This may improve detection accuracy of the system as well as improve the perceived quality of the sound that the therapy device user experiences.

In some embodiments, masks may be designed to have unique acoustic sound response characteristics. For example, a unique sound resonator or unique characteristic dimensions may be designed within the mask or conduit to permit easier differentiation of the acoustic reflection data of each mask.

In some embodiments, autocorrelation (i.e., the inverse Fourier Transform of the power spectrum) may be implemented rather than cepstrum analysis.

In further embodiments of the present technology, acoustic reflections may be analyzed to identify particular characteristics of masks in addition to identification of type. For example, system response data may be utilized to identify characteristics of masks and conduits. The characteristics may include: diameter, construction materials, volume of air cavities, overall configurations of the masks and conduits, etc.

**5.9 GLOSSARY**

For the purposes of the present technology disclosure, in certain forms of the present technology, one or more of the following definitions may apply. In other forms of the present technology, alternative definitions may apply.

*5.9.1 General*

*Air:* In certain forms of the present technology, air may be taken to mean atmospheric air, and in other forms of the present technology air may be taken to mean some other combination of breathable gases, e.g. atmospheric air enriched with oxygen.

*Ambient:* In certain forms of the present technology, the term ambient will be taken to mean (i) external of the respiratory therapy system or patient, and (ii) immediately surrounding the respiratory therapy system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

*Leak:* The word leak will be taken to be an unintended flow of air. In one example, leak may occur as the result of an incomplete seal between a mask and a patient's face. In another example leak may occur in a swivel elbow to the ambient.

*Patient:* A person, whether or not they are suffering from a respiratory condition.

*Pressure:* Force per unit area. Pressure may be expressed in a range of units, including $cmH_2O$, $g-f/cm^2$ and hectopascal. 1 $cmH_2O$ is equal to 1 $g-f/cm^2$ and is approximately 0.98 hectopascal. In this specification, unless otherwise stated, pressure is given in units of $cmH_2O$.

*Respiratory Pressure Therapy (RPT):* The application of a supply of air to an entrance to the airways at a treatment pressure that is typically positive with respect to atmosphere.

*Seal:* May be a noun form ("a seal") which refers to a structure, or a verb form ("to seal") which refers to the effect. Two elements may be constructed and/or arranged to 'seal' or to effect 'sealing' therebetween without requiring a separate 'seal' element *per se.*

*5.9.2 Patient interface*

*Plenum chamber:* a mask plenum chamber will be taken to mean a portion of a patient interface having walls at least partially enclosing a volume of space, the volume having air therein pressurised above atmospheric pressure in use. A shell may form part of the walls of a mask plenum chamber.

*Shell:* A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

*Vent:* (noun): A structure that allows a flow of *air* from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

## 5.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

The term "about" is used herein to refer to quantities that vary by as much as 30%, preferably by as much as 20%, and more preferably by as much as 10% to a reference quantity. The use of the word 'about' to qualify a number is merely an express indication that the number is not to be construed as a precise value.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced. Thus, throughout this specification, unless the context requires otherwise, the words "comprise", "comprises" and "comprising" will be understood to imply the inclusion of a stated step or element or group of steps or elements but not the exclusion of any other step or element or group of steps or elements. Any one of the terms: "including" or "which includes" or "that includes" as used herein is also an open term that also means including at least the elements/features that follow the term, but not excluding others. Thus, "including" is synonymous with and means "comprising".

The various methods or processes outlined herein may be coded as software that is executable on one or more processors that employ any one of a variety of operating systems or platforms. Additionally, such software may be written using any of a number of suitable programming languages and/or programming or scripting tools, and also may be compiled as executable machine language code or intermediate code that is executed on a framework or virtual machine.

In this respect, various inventive concepts may be embodied as a processor readable medium or computer readable storage medium (or multiple such storage media) (e.g., a computer memory, one or more floppy discs, compact discs, optical discs, magnetic tapes, flash memories, circuit configurations in Field Programmable Gate Arrays or other semiconductor devices, or other non-transitory medium or tangible computer storage medium) encoded with one or more programs or processor control instructions that, when executed on one or more computers or other processors, perform methods that implement the various embodiments of the technology discussed above. The computer readable medium or media can be transportable, such that the program or programs stored thereon can be loaded onto one or more different computers or other processors to implement various aspects of the present technology as discussed above.

The terms "program" or "software" are used herein in a generic sense to refer to any type of computer code or set of computer-executable instructions that can be employed to program a computer or other processor to implement various aspects of embodiments as discussed above. Additionally, it should be appreciated that according to one aspect, one or more computer programs that when executed perform methods of the present technology need not reside on a single computer or processor, but may be distributed in a modular fashion amongst a number of different computers or processors to implement various aspects of the present technology. For example, some versions of the present technology may include a server with access to any of the computer readable or processor-readable mediums as described herein. The server may be configured to receive requests for downloading the processor-control instructions or processor-executable instructions of the medium to an electronic device, such as a smart mobile phone or smart speaker, over a network such as a communications network, an internet or the Internet. Thus, the electronic device may also include such a medium to execute the instructions of the medium. Similarly, the present technology may be implemented as a method of a server having access to any of the mediums described herein. The method(s) may include receiving, at the server, a request for downloading the processor-executable instructions of the medium to an electronic device over the network; and transmitting the instructions of the medium to the electronic device in response to the request. Optionally, the server may have access to the medium to execute the instructions of the medium.

Computer-executable instructions may be in many forms, such as program modules, executed by one or more computers or other devices. Generally, program modules include routines, programs, objects, components, data structures, etc. that perform particular tasks or implement particular abstract data types. Typically the functionality of the program modules may be combined or distributed as desired in various embodiments.

Also, data structures may be stored in computer-readable media in any suitable form. For simplicity of illustration, data structures may be shown to have fields that are related through location in the data structure. Such relationships may likewise be achieved by assigning storage for the fields with locations in a computer-readable medium that convey relationship between the fields. However, any suitable mechanism may be used to establish a relationship between information in fields of a data structure, including through the use of pointers, tags or other mechanisms that establish relationship between data elements.

Although the technology herein has been described with reference to particular examples, it is to be understood that these examples are merely illustrative of the principles and applications of the technology. For example, although the acoustic generator(s) and acoustic monitoring techniques are described herein in particular examples concerning the use, and component(s) of, RPT device(s), it will be understood that such acoustic generator(s) and acoustic monitoring techniques may be similarly implemented with the component(s) of any respiratory therapy (RT) device such as a high flow therapy (HFT) device that provides a controlled flow of air at therapeutic flow levels through a patient interface. Thus, the HFT device is similar to a pressure-controlled RPT device but configured with a controller adapted for flow control. In such examples, the acoustic generator(s) may be configured for measuring a gas characteristic associated with the high flow therapy generated by the HFT device and may be integrated to sample the gas flow of a patient circuit, a conduit coupler thereof, and/or a patient interface of the HFT device. Thus, the HFT device may optionally include an acoustic receiver, as well as the processing techniques for acoustic analysis as described herein, for receiving the acoustic/sound signal generated by the acoustic generator implemented HFT device.

In some instances herein, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

### 5.11 REFERENCE SIGNS LIST

| patient | 1000 |
|---|---|
| trace | 1050 |
| peak | 1060 |
| trough | 1070 |
| bed partner | 1100 |
| system | 1105 |
| conduit | 1110 |
| system component | 1120 |
| patient respiratory airways | 1130 |
| RPT device / humidifier | 1140 |
| microphone | 1150 |
| dampening structure | 1160 |
| method | 1200 |
| step | 1210 |
| step | 1220 |

(continued)

| step | 1230 |
|---|---|
| step | 1240 |
| step | 1250 |
| step | 1260 |
| graph | 1300 |
| graph | 1350 |
| patient interface | 3000 |
| seal - forming structure | 3100 |
| plenum chamber | 3200 |
| structure | 3300 |
| vent | 3400 |
| connection port | 3600 |
| forehead support | 3700 |
| RPT device | 4000 |
| external housing | 4010 |
| upper portion | 4012 |
| portion | 4014 |
| panel | 4015 |
| chassis | 4016 |
| handle | 4018 |
| pneumatic block | 4020 |
| inlet air filter | 4112 |
| inlet muffler | 4122 |
| outlet muffler | 4124 |
| pressure generator | 4140 |
| blower | 4142 |
| air circuit | 4170 |
| electrical components | 4200 |
| PCBA | 4202 |
| electrical power supply | 4210 |
| input devices | 4220 |
| central controller | 4230 |
| therapy device controller | 4240 |
| protection circuits | 4250 |
| memory | 4260 |
| pressure sensor microphone | 4270 |
| data communication interface | 4280 |
| remote external communication network | 4282 |
| local external communication network | 4284 |
| remote external device | 4286 |

(continued)

| | |
|---|---|
| local external device | 4288 |
| output device | 4290 |
| algorithms | 4300 |
| humidifier | 5000 |
| humidifier inlet | 5002 |
| humidifier outlet | 5004 |
| humidifier base | 5006 |
| water reservoir | 5110 |
| conductive portion | 5120 |
| humidifier reservoir dock | 5130 |
| locking lever | 5135 |
| water level indicator | 5150 |
| humidifier transducer | 5210 |
| air pressure sensor | 5212 |
| air flow rate transducer | 5214 |
| temperature sensor | 5216 |
| humidity sensor | 5218 |
| heating element | 5240 |
| controller | 5250 |
| system | 7000 |
| conduit | 7010 |
| mask | 7020 |
| system | 7030 |
| RPT device / humidifier | 7040 |
| microphone | 7050 |
| input signal component | 8010 |
| reflection component | 8020 |
| acoustic generator | 8500 |

## Claims

1. A device for generating a respiratory therapy, the device comprising:

   a pressure generator (4140) configured to generate a supply of pressurized air from an outlet along an air circuit (4170) to a patient interface (3000);
   a sensor (4270) configured to generate a sound signal representing a sound of the pressure generator in the air circuit;
   a dampening structure (1106) configured to reduce reflection of sound from the pressure generator along the air circuit; and
   a controller configured to:
   process the sound signal so as to identify the patient interface and / or the air circuit.

2. The device of claim 1 wherein the dampening structure (1106) is formed by a pass-through dampening conduit configured to change acoustic impedance between the air circuit (4170) and a cavity of a housing of the pressure generator (4140).

3. The device of claim 2 wherein the dampening structure (1106) defines a cross-section of a passage through the pass-through dampening conduit such that the cross-section expands, due to a profile of an internal surface of the pass-through dampening conduit, along a path of the passage.

4. The device of claim 3 wherein the cross-section gradually expands as the cross-section is more distant from a patient interface (3000) end of the air circuit (4170).

5. The device of any one of claims 1 to 4 wherein the device comprises a waveguide at least formed by the outlet and the air circuit (4170), and wherein the dampening structure (1106) is located along the waveguide between the sensor (4270) and the outlet, and wherein the sensor is located along the waveguide between the dampening structure and the air circuit.

6. The device of any one of claims 1 to 5, wherein the dampening structure (1106) is formed by a horn.

7. The device of claim 6, wherein the horn has a conical profile.

8. The device of any one of claims 1 to 7, wherein the controller is configured to:

process the sound signal to obtain a cepstrum, wherein the processing comprises flattening a spectrum of the sound signal;
separate an acoustic signature from the cepstrum;
compare the acoustic signature to a set of predetermined acoustic signatures corresponding to respective components; and
identify the patient interface (3000) and / or the air circuit (4170) based on the comparison of the acoustic signature to the set.

9. The device of claim 8 wherein the controller is further configured to adjust a control parameter for operation of the pressure generator (4140) based on the identified patient interface (3000) and/or air circuit (4170).

10. The device of any one of claims 1 to 7 further comprising a computer-readable medium having encoded thereon computer-readable instructions that when executed by a processor of the controller of the device cause the processor to perform a method for identifying a component of an airpath coupled to the respiratory therapy device wherein:

(a) the method comprises:

processing a sound signal representing a sound in the airpath to obtain a cepstrum, wherein the processing comprises flattening a spectrum of the sound signal;
separating an acoustic signature from the cepstrum;
comparing the acoustic signature to a set of predetermined acoustic signatures corresponding to respective components; and
identifying the component based on the comparison of the acoustic signature to the set; and/or

(b) the method comprises:

processing a sound signal representing a sound in the airpath to obtain a cepstrum;
separating an acoustic signature from the cepstrum;
repeating the processing and separating at least once to generate a plurality of acoustic signatures,
combining the plurality of acoustic signatures into a combined acoustic signature,
comparing the combined acoustic signature to a set of predetermined acoustic signatures corresponding to respective components; and
identifying the component based on the comparison of the acoustic signature to the set.

11. The device of any one of claims 1 to 7, wherein the controller is configured to:

process the sound signal to obtain a cepstrum;
separate an acoustic signature from the cepstrum;

repeat the processing and separating at least once to generate multiple acoustic signatures,
combine the multiple acoustic signatures into a combined acoustic signature,
compare the combined acoustic signature to a set of predetermined acoustic signatures corresponding to respective components; and
identify the patient interface (3000) and / or the air circuit (4170) based on the comparison of the acoustic signature to the set.

12. The device of claim 11 wherein to combine the multiple acoustic signatures the controller is configured to align one or more of the multiple acoustic signatures with the combined acoustic signature.

13. The device of any one of claims 11 to 12, wherein to combine the multiple acoustic signatures, the controller is configured to average the multiple acoustic signatures.

14. The device of any one of claims 11 to 13, wherein the controller is further configured to adjust a control parameter for operation of the pressure generator (4140) based on the identified patient interface (3000) and/or air circuit (4170).


**Patentansprüche**

1. Vorrichtung zum Erzeugen einer Atemtherapie, wobei die Vorrichtung Folgendes aufweist:

einen Druckerzeuger (4140), der dazu konfiguriert ist, eine Zufuhr von Druckluft von einem Auslass entlang eines Luftkreislaufs (4170) zu einer Patientenschnittstelle (3000) zu erzeugen;
einen Sensor (4270), der dazu konfiguriert ist, ein Geräuschsignal zu erzeugen, das ein Geräusch des Druckerzeugers in dem Luftkreislauf repräsentiert;
eine Dämpfungsstruktur (1106), die dazu konfiguriert ist, eine Reflexion eines Geräusches von dem Druckerzeuger entlang des Luftkreislaufs zu verringern; und
eine Steuereinheit, die dazu konfiguriert ist:
das Geräuschsignal zu verarbeiten, um die Patientenschnittstelle und/oder den Luftkreislauf zu identifizieren.

2. Vorrichtung nach Anspruch 1, wobei die Dämpfungsstruktur (1106) von einer Durchgangs-Dämpfungsleitung gebildet ist, die dazu konfiguriert ist, die akustische Impedanz zwischen dem Luftkreislauf (4170) und einer Höhlung eines Gehäuses des Druckerzeugers (4140) zu ändern.

3. Vorrichtung nach Anspruch 2, wobei die Dämpfungsstruktur (1106) einen Querschnitt eines Durchgangs durch die Durchgangs-Dämpfungsleitung derart definiert, dass sich der Querschnitt aufgrund eines Profils einer Innenoberfläche der Durchgangs-Dämpfungsleitung entlang eines Pfads des Durchgangs ausdehnt.

4. Vorrichtung nach Anspruch 3, wobei sich der Querschnitt mit zunehmender Entfernung des Querschnitts von einem Patientenschnittstellen(3000)-Ende des Luftkreislaufs (4170) allmählich ausdehnt.

5. Vorrichtung nach einem der Ansprüche 1 bis 4, wobei die Vorrichtung einen Wellenleiter aufweist, der zumindest von dem Auslass und dem Luftkreislauf (4170) gebildet wird, und wobei sich die Dämpfungsstruktur (1106) entlang des Wellenleiters zwischen dem Sensor (4270) und dem Auslass befindet und wobei sich der Sensor entlang des Wellenleiters zwischen der Dämpfungsstruktur und dem Luftkreislauf befindet.

6. Vorrichtung nach einem der Ansprüche 1 bis 5, wobei die Dämpfungsstruktur von (1106) von einem Trichter gebildet wird.

7. Vorrichtung nach Anspruch 6, wobei der Trichter ein konisches Profil aufweist.

8. Vorrichtung nach einem der Ansprüche 1 bis 7, wobei die Steuereinheit zu Folgendem konfiguriert ist:

Verarbeiten des Klangsignals, um ein Cepstrum zu erhalten, wobei das Verarbeiten ein Glätten eines Spektrums des Klangsignals umfasst;
Trennen einer akustischen Signatur von dem Cepstrum;
Vergleichen der akustischen Signatur mit einem Satz vorgegebener akustischer Signaturen, die jeweiligen Komponenten entsprechen; und

Identifizieren der Patientenschnittstelle (3000) und/oder des Luftkreislaufs (4170) basierend auf dem Vergleich der akustischen Signatur mit dem Satz.

9. Vorrichtung nach Anspruch 8, wobei die Steuereinheit ferner dazu konfiguriert ist, einen Steuerparameter für einen Betrieb des Druckerzeugers (4140) basierend auf der identifizierten Patientenschnittstelle (3000) und/oder dem identifizierten Luftkreislauf (4170) anzupassen.

10. Vorrichtung nach einem der Ansprüche 1 bis 7, ferner aufweisend ein computerlesbares Medium, auf dem computerlesbare Anweisungen codiert sind, die, wenn sich von einem Prozessor der Steuereinheit der Vorrichtung ausgeführt werden, den Prozessor veranlassen, ein Verfahren zum Identifizieren einer Komponente eines an die Atemtherapievorrichtung gekoppelten Luftpfads durchzuführen, wobei:

(a) das Verfahren Folgendes umfasst:

Verarbeiten eines Geräuschsignals, das ein Geräusch in dem Luftpfad repräsentiert, um ein Cepstrum zu erhalten, wobei das Verarbeiten ein Glätten eines Spektrums des Klangsignals umfasst;
Trennen einer akustischen Signatur von dem Cepstrum;
Vergleichen der akustischen Signatur mit einem Satz vorgegebener akustischer Signaturen, die jeweiligen Komponenten entsprechen; und
Identifizieren der Komponente basierend auf dem Vergleich der akustischen Signatur mit dem Satz; und/oder

(b) das Verfahren Folgendes umfasst:

Verarbeiten eines Geräuschsignals, das ein Geräusch in dem Luftpfad repräsentiert, um ein Cepstrum zu erhalten;
Trennen einer akustischen Signatur von dem Cepstrum;
Wiederholen der Verarbeitung und der Trennung zumindest einmal, um eine Vielzahl von akustischen Signaturen zu erzeugen,
Kombinieren der Vielzahl von akustischen Signaturen zu einer kombinierten akustischen Signatur,
Vergleichen der kombinierten akustischen Signatur mit einem Satz von vorgegebenen akustischen Signaturen, die jeweiligen Komponenten entsprechen; und
Identifizieren der Komponente basierend auf dem Vergleich der akustischen Signatur mit dem Satz.

11. Vorrichtung nach einem der Ansprüche 1 bis 7, wobei die Steuereinheit zu Folgendem konfiguriert ist:

Verarbeiten des Geräuschsignals, um ein Cepstrum zu erhalten;
Trennen einer akustischen Signatur von dem Cepstrum;
Wiederholen der Verarbeitung und der Trennung zumindest einmal, um mehrere akustischen Signaturen zu erzeugen,
Kombinieren der mehreren akustischen Signaturen zu einer kombinierten akustischen Signatur,
Vergleichen der kombinierten akustischen Signatur mit einem Satz von vorgegebenen akustischen Signaturen, die jeweiligen Komponenten entsprechen; und
Identifizieren der Patientenschnittstelle (3000) und/oder des Luftkreislaufs (4170) basierend auf dem Vergleich der akustischen Signatur mit dem Satz.

12. Vorrichtung nach Anspruch 11, wobei die Steuereinheit zum Kombinieren der mehreren akustischen Signaturen dazu konfiguriert ist, eine oder mehrere der mehreren akustischen Signaturen mit der kombinierten akustischen Signatur zur Deckung zu bringen.

13. Vorrichtung nach einem der Ansprüche 11 bis 12, wobei die Steuereinheit zum Kombinieren der mehreren akustischen Signaturen dazu konfiguriert ist, die mehreren akustischen Signaturen zu mitteln.

14. Vorrichtung nach einem der Ansprüche 11 bis 13, wobei die Steuereinheit ferner dazu konfiguriert ist, einen Steuerparameter für einen Betrieb des Druckerzeugers (4140) basierend auf der identifizierten Patientenschnittstelle (3000) und/oder dem identifizierten Luftkreislauf (4170) anzupassen.

**EP 3 962 560 B1**

## Revendications

1. Dispositif pour générer une thérapie respiratoire, le dispositif comprenant :

    un générateur de pression (4140) configuré pour générer une alimentation en air comprimé à partir d'une sortie le long d'un circuit d'air (4170) vers une interface de patient (3000) ;
    un capteur (4270) configuré pour générer un signal sonore représentant un son du générateur de pression dans le circuit d'air ;
    une structure d'amortissement (1106) configurée pour réduire la réflexion du son provenant du générateur de pression le long du circuit d'air ; et
    un dispositif de commande configuré pour :
    traiter le signal sonore de façon à identifier l'interface patient et/ou le circuit d'air.

2. Dispositif selon la revendication 1, dans lequel la structure d'amortissement (1106) est formée par un conduit d'amortissement traversant configuré pour modifier l'impédance acoustique entre le circuit d'air (4170) et une cavité d'un boîtier du générateur de pression (4140).

3. Dispositif selon la revendication 2, dans lequel la structure d'amortissement (1106) définit une section transversale d'un passage à travers le conduit d'amortissement traversant de telle sorte que la section transversale s'agrandit, en raison d'un profil d'une surface interne du conduit d'amortissement traversant, le long d'un trajet du passage.

4. Dispositif selon la revendication 3, dans lequel la section transversale s'agrandit progressivement à mesure que la section transversale est plus éloignée d'une extrémité d'interface patient (3000) du circuit d'air (4170).

5. Dispositif selon l'une quelconque des revendications 1 à 4, dans lequel le dispositif comprend un guide d'ondes au moins formé par la sortie et le circuit d'air (4170), et dans lequel la structure d'amortissement (1106) est située le long du guide d'ondes entre le capteur (4270) et la sortie, et dans lequel le capteur est situé le long du guide d'ondes entre la structure d'amortissement et le circuit d'air.

6. Dispositif selon l'une quelconque des revendications 1 à 5, dans lequel la structure d'amortissement (1106) est formée par un pavillon.

7. Dispositif selon la revendication 6, dans lequel le pavillon a un profil conique.

8. Dispositif selon l'une quelconque des revendications 1 à 7, dans lequel le dispositif de commande est configuré pour :

    traiter le signal sonore pour obtenir un cepstre, le traitement comprenant l'aplatissement d'un spectre du signal sonore ;
    séparer une signature acoustique du cepstre ;
    comparer la signature acoustique à un ensemble de signatures acoustiques prédéterminées correspondant aux composants respectifs ; et
    identifier l'interface patient (3000) et/ou le circuit d'air (4170) sur la base de la comparaison de la signature acoustique à l'ensemble.

9. Dispositif selon la revendication 8, dans lequel le dispositif de commande est en outre configuré pour ajuster un paramètre de commande pour le fonctionnement du générateur de pression (4140) sur la base de l'interface patient (3000) et/ou du circuit d'air (4170) identifié.

10. Dispositif selon l'une quelconque des revendications 1 à 7, comprenant en outre un support lisible par ordinateur sur lequel sont codées des instructions lisibles par ordinateur qui, lorsqu'elles sont exécutées par un processeur du dispositif de commande du dispositif, amènent le processeur à exécuter un procédé pour identifier un composant d'un trajet d'air raccordé à l'appareil de thérapie respiratoire dans lequel :

    (a) le procédé comprend :

        un traitement d'un signal sonore représentant un son dans le trajet d'air pour obtenir un cepstre, le traitement comprenant l'aplatissement d'un spectre du signal sonore ;
        une séparation d'une signature acoustique du cepstre ;

une comparaison de la signature acoustique à un ensemble de signatures acoustiques prédéterminées correspondant aux composants respectifs ; et
une identification du composant sur la base de la comparaison de la signature acoustique à l'ensemble ;

et/ou
(b) le procédé comprend :

un traitement d'un signal sonore représentant un son dans le trajet d'air pour obtenir un cepstre ;
une séparation d'une signature acoustique du cepstre ;
une répétition du traitement et de la séparation au moins une fois pour générer une pluralité de signatures acoustiques,
une combinaison de la pluralité de signatures acoustiques en une signature acoustique combinée,
une comparaison de la signature acoustique combinée à un ensemble de signatures acoustiques prédéterminées correspondant aux composants respectifs ; et
une identification du composant sur la base de la comparaison de la signature acoustique à l'ensemble.

11. Dispositif selon l'une quelconque des revendications 1 à 7, dans lequel le dispositif de commande est configuré pour :

traiter le signal sonore pour obtenir un cepstre ;
séparer une signature acoustique du cepstre ;
répéter le traitement et la séparation au moins une fois pour générer des signatures acoustiques multiples,
combiner les multiples signatures acoustiques en une signature acoustique combinée,
comparer la signature acoustique combinée à un ensemble de signatures acoustiques prédéterminées correspondant aux composants respectifs ; et
identifier l'interface patient (3000) et/ou le circuit d'air (4170) sur la base de la comparaison de la signature acoustique à l'ensemble.

12. Dispositif selon la revendication 11 dans lequel, pour combiner les multiples signatures acoustiques, le dispositif de commande est configuré pour aligner une ou plusieurs des multiples signatures acoustiques avec la signature acoustique combinée.

13. Dispositif selon l'une quelconque des revendications 11 à 12, dans lequel, pour combiner les multiples signatures acoustiques, le dispositif de commande est configuré pour établir la moyenne des multiples signatures acoustiques.

14. Dispositif selon l'une quelconque des revendications 11 à 13, dans lequel le dispositif de commande est en outre configuré pour ajuster un paramètre de commande pour le fonctionnement du générateur de pression (4140) sur la base de l'interface patient (3000) et/ou du circuit d'air (4170) identifié.

**FIG. 1A**

FIG. 1B

FIG. 1C

Nasal cavity
Oral cavity
Larynx
Vocal folds
Oesophagus
Trachea
Bronchus
Lung
Heart
Diaphragm
Alveolar sacs

# FIG. 2

Copyright 2012 ResMed Limited

**FIG. 3**

4000

4015
4018
4220
4012
4202
4142
4016
4210
4014

4112
4015

Superior
Posterior
Anterior
Inferior

4100, 4020
4200
4010

**FIG. 4A**

4110 — 4112 Air inlet filter ← Supplementary O₂

Pneumatic block

4120 — 4122 Inlet muffler

4270 — Transducer(s)

4140 — | 4142 Blower | 4144 Motor |

4180

4020

4120 — 4124 Outlet muffler

4270 — Transducer(s)

Upstream

↕

Downstream

4160 — Anti-spillback valve

5000 — Humidifier

4270 — Transducer(s)

4180

4170 — Air circuit / delivery tube ← Supplementary O₂

4270 — Transducer(s)

4180

3000 — Patient Interface ← Supplementary O₂

# FIG. 4B

**FIG. 5A**

**FIG. 5B**

**FIG. 6**

EP 3 962 560 B1

**FIG. 7**

FIG. 8

EP 3 962 560 B1

FIG. 9

quefrency

Legend:
- Ultra Mirage, 10000rpm
- Ultra Mirage, 15000rpm
- Mirage Quattro, 10000rpm
- Mirage Quattro, 15000rpm
- Swift II, 10000rpm
- Swift II, 15000rpm

FIG. 10

**FIG. 11**

**FIG. 11A**

**FIG. 11B**

1200

1210
Compute output cepstrum

1220
Separate acoustic signature from cepstrum

1230
Incorporate acoustic signature into combined acoustic signature

1240
Finished?

1260
N → Await next synchronised window

Y

1250
Compare combined acoustic signature with data set to identify component

FIG. 12

**FIG. 13**

EP 3 962 560 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2019901502
- US 4944310 A
- WO 2010091462 A1
- AU 2014271343 A1
- AU 2013201312 A1
- WO 2019006496 A1
- US 7866944 B
- US 8638014 B
- US 8636479 B
- WO 2013020167 A
- WO 2010091462 A

**Non-patent literature cited in the description**

- **JOHN B. WEST**. Respiratory Physiology. Lippincott Williams & Wilkins, 2012
- **CHILDERS et al.** The Cepstrum: A Guide to Processing. *Proceedings of the IEEE*, October 1977, vol. 65 (10)
- **RANDALL RB**. Frequency Analysis. Bruel & Kjaer, 1977, 344